(19) Europäisches Patentamt
European Patent Office
Office européen des brevets

(11) EP 2 507 391 B1

(12) **EUROPEAN PATENT SPECIFICATION**

(45) Date of publication and mention of the grant of the patent:
**17.08.2016 Bulletin 2016/33**

(21) Application number: **10818076.1**

(22) Date of filing: **02.12.2010**

(51) Int Cl.:
***C12Q 1/68*** *(2006.01)*

(86) International application number:
**PCT/IB2010/003490**

(87) International publication number:
**WO 2011/067678 (09.06.2011 Gazette 2011/23)**

(54) **COMPOSITIONS AND METHODS FOR PERFORMING HYBRIDIZATIONS WITH NO DENATURATION**

ZUSAMMENSETZUNGEN UND VERFAHREN ZUR HYBRIDISIERUNG OHNE DENATURIERUNG

COMPOSITIONS ET PROCÉDÉS POUR RÉALISER DES HYBRIDATIONS SANS DÉNATURATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2009 US 265966 P**

(43) Date of publication of application:
**10.10.2012 Bulletin 2012/41**

(73) Proprietor: **Dako Denmark A/S**
**2600 Glostrup (DK)**

(72) Inventor: **MATTHIESEN, Steen, H.**
**3400 Hillerod (DK)**

(74) Representative: **Grünecker Patent- und Rechtsanwälte**
**PartG mbB**
**Leopoldstraße 4**
**80802 München (DE)**

(56) References cited:

**WO-A1-91/02088**
**WO-A1-92/07956**
**WO-A1-94/02639**
**WO-A1-2004/097043**
**WO-A1-2009/144581**
**WO-A1-2010/097655**
**WO-A1-2010/097707**
**WO-A2-2009/074154**

- **SEN ANJANA ET AL: "On the stability of peptide nucleic acid duplexes in the presence of organic solvents", NUCLEIC ACIDS RESEARCH, vol. 35, no. 10, May 2007 (2007-05), pages 3367-3374, XP002638105, ISSN: 0305-1048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 507 391 B1

**Description**

This application claims priority to U.S. Provisional Application No. 61/265,966, filed December 2, 2009.

**FIELD OF THE INVENTION**

The present invention relates to methods for eliminating the denaturation step from hybridization applications. In one embodiment, the present invention can be used for the *in vivo, in vitro,* and *in situ* molecular examination of DNA and RNA. In particular, the invention can be used for the molecular examination of DNA and RNA in the fields of cytology, histology, and molecular biology. In other embodiments, the present invention can be used for *in situ* hybridization (ISH) applications.

**BACKGROUND AND DESCRIPTION**

Double stranded nucleic acid molecules *(i.e.,* DNA (deoxyribonucleic acid), DNA/RNA (ribonucleic acid) and RNA/RNA) associate in a double helical configuration. This double helix structure is stabilized by hydrogen bonding between bases on opposite strands when bases are paired in one particular way (A+T/U or G+C) and hydrophobic bonding among the stacked bases. Complementary base paring (hybridization) is central to all processes involving nucleic acid.

In a basic example of hybridization, nucleic acid probes or primers are designed to bind, or "hybridize," with a target nucleic acid, for example, DNA or RNA in a sample. One type of hybridization application, *in situ* hybridization (ISH), includes hybridization to a target in a specimen wherein the specimen may be *in vivo, in situ,* or for example, fixed or adhered to a glass slide. The probes may be labeled to make identification of the probe-target hybrid possible by use of a fluorescence or bright field microscope/scanner. Such labeled probes can be used, for example, to detect genetic abnormalities in a target sequence, providing valuable information about, *e.g.,* prenatal disorders, cancer, and other genetic or infectious diseases.

In order for the probes or primers to bind to the target nucleic acid in the sample, complementary strands of nucleic acid must be separated. This strand separation step, termed "denaturation," typically requires aggressive conditions to disrupt the hydrogen and hydrophobic bonds in the double helix. Once the complementary strands of nucleic acid have been separated, a "renaturation" or "reannealing" step allows the primers or probes to bind to the target nucleic acid in the sample. This step is also sometimes referred to as the "hybridization" step.

Traditional hybridization experiments, such as ISH assays, use high temperatures (*e.g.*, 95°C to 100°C) and/or formamide-containing solutions to denature doubled stranded nucleic acid. However, these methods have significant drawbacks.

For example, heat can be destructive to the structure of the nucleic acid itself because the phosphodiester bonds may be broken at high temperatures, leading to a collection of broken single stranded nucleic acids. In addition, heat can lead to complications when small volumes are used, since evaporation of aqueous buffers is difficult to control.

Formamide-containing solutions are often used to overcome some of the problems associated with heat-denaturation. Formamide disrupts base pairing by displacing loosely and uniformly bound hydrate molecules and by causing "formamidation" of the Watson-Crick binding sites. Thus, formamide has a destabilizing effect on double stranded nucleic acids and analogs, allowing denaturation to occur at lower temperatures. However, although formamide lowers the melting temperature (Tm) of double-stranded nucleic acid, it also significantly prolongs the renaturation time, as compared to aqueous denaturation solutions without formamide.

In addition, formamide has disadvantages beyond a long processing time. Formamide is a toxic, hazardous material, subject to strict regulations for use and waste. Furthermore, the use of a high concentration of formamide appears to cause morphological destruction of cellular, nuclear, and/or chromosomal structure.

Thus, a need exists for overcoming the drawbacks associated with the denaturation step of hybridization applications. By addressing this need, the present invention provides several potential advantages over prior art hybridization applications, such as faster hybridization times, lower hybridization temperatures, and less toxic hybridization solvents.

**SUMMARY OF THE INVENTION**

It is an object of the present invention to provide methods which result in hybridization applications having at least one of the following advantages over prior art hybridization applications: lower background, lower evaporation of reagent, preservation of sample morphology, simpler procedure, faster procedure, easier automation, and safer reagents. The present invention achieves those objectives by eliminating the denaturation step.

Accordingly, one aspect of the invention relates to a method of hybridizing nucleic acid sequences in situ in a

sample on a slide without a denaturation step and on an automated system comprising:

- providing a first nucleic acid sequence,
- providing a second nucleic acid sequence,
- providing a hybridization composition comprising an effective amount of at least one polar aprotic solvent, and
- combining the first and the second nucleic acid sequence and the hybridization composition on the automated system for at least a time period sufficient to hybridize the first and second nucleic acid sequences,

wherein the polar aprotic solvent is not dimethyl sulfoxide (DMSO).

A further aspect of the invention relates to a method of hybridizing nucleic acid sequences in situ in a sample on a slide without a denaturation step and on an automated system comprising:

- providing a first nucleic acid sequence, and

- applying a hybridization composition comprising a second nucleic acid sequence and an effective amount of at least one polar aprotic solvent for at least a time period sufficient to hybridize the first and second nucleic acid sequences, wherein the polar aprotic solvent is not dimethyl sulfoxide (DMSO).

The methods of the invention are applicable to any hybridization technique. The methods of the invention are also applicable to any molecular system that hybridizes or binds using base pairing, such as, for example, DNA, RNA, PNA, LNA, and synthetic and natural analogs thereof.

The nucleic acid hybridization method of the present invention may be used for the *in vivo* or *in vitro* analysis of genomic DNA, chromosomes, chromosome fragments, genes, and chromosome aberrations such as translocations, deletions, amplifications, insertions, mutations, or inversions associated with a normal condition or a disease. Further, the methods are useful for detection of infectious agents as well as changes in levels of expression of RNA, e.g., mRNA and its complementary DNA (cDNA).

Other uses include the *in vivo, in vitro,* or *in situ* analysis of messenger RNA (mRNA), viral RNA, viral DNA, small interfering RNA (siRNA), small nuclear RNA (snRNA), non-coding RNA (ncRNA, *e.g.,* tRNA and rRNA), transfer messenger RNA (tmRNA), micro RNA (miRNA), piwi-interacting RNA (piRNA), long noncoding RNA, small nucleolar RNA (snoRNA), antisense RNA, double-stranded RNA (dsRNA), methylations and other base modifications, single nucleotide polymorphisms (SNPs), copy number variations (CNVs), and nucleic acids labeled with, e.g., radioisotopes, fluorescent molecules, biotin, digoxigenin (DIG), or antigens, alone or in combination with unlabeled nucleic acids.

The nucleic acid hybridization method of the present invention are useful for *in vivo, in vitro*, or *in situ* analysis of nucleic acids using techniques such as PCR, *in situ* PCR, northern blot, Southern blot, flow cytometry, autoradiography, fluorescence microscopy, chemiluminescence, immunohistochemistry, virtual karyotype, gene assay, DNA microarray (*e.g.*, array comparative genomic hybridization (array CGH)), gene expression profiling, Gene ID, Tiling array, gel electrophoresis, capillary electrophoresis, and *in situ* hybridizations such as FISH, SISH, CISH. In one embodiment, the methods of the invention are useful for nucleic acid hybridization applications, with the proviso that such applications do not include amplification of the nucleic acid such as, *e.g.,* by PCR, *in situ* PCR, etc.

The methods of the invention may be used on *in vitro* and *in vivo* samples such as bone marrow smears, blood smears, paraffin embedded tissue preparations, enzymatically dissociated tissue samples, bone marrow, amniocytes, cytospin preparations, imprints, etc.

The invention provides methods for hybridizing at least one molecule to a target in situ in a sample on a slide without a denaturation step and on an automated system. In certain embodiments, the method of the invention significantly reduces the background levels without the need for blocking agents, and without the need for overnight hybridization in formamide-containing buffers. Thus, the invention may, for example, eliminate the use of, or reduce the dependence on formamide. Accordingly, in some aspects, the present invention overcomes the major toxicity issue and the time consuming renaturation step associated with the use of formamide traditional hybridization assays.

One disclosure of the application is a composition or solution for use in hybridization applications. Compositions for use according to this disclosure include an aqueous composition comprising at least one nucleic acid sequence and at least one polar aprotic solvent in an amount effective to denature double-stranded nucleotide sequences. An amount effective to denature double-stranded nucleotide sequences is an amount that enables hybridization. For example, one way to test for whether the amount of polar aprotic solvent is effective to enable hybridization is to determine whether the polar aprotic solvent, when used in the hybridization methods and compositions described herein, such as example 1, yield a detectable signal and/or an amplified nucleic acid product.

Non-limiting examples of effective amounts of polar aprotic solvents include, e.g., about 1% to about 95% (v/v). In some embodiments, the concentration of polar aprotic solvent is 5% to 60% (v/v). In other embodiments, the concentration of polar aprotic solvent is 10% to 60% (v/v). In still other embodiments, the concentration of polar aprotic solvent

is 30% to 50% (v/v). Concentrations of 1% to 5%, 5% to 10%, 10%, 10% to 20%, 20% to 30%, 30% to 40%, 40% to 50%, or 50% to 60% (v/v) are also suitable. In some embodiments, the polar aprotic solvent will be present at a concentration of 0.1%, 0.25%, 0.5%, 1%, 2%, 3%, 4%, or 5% (v/v). In other embodiments, the polar aprotic solvent will be present at a concentration of 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, or 20% (v/v).

The application further discloses the aqueous composition comprising a polar aprotic solvent having reduced toxicity. For example, a less-toxic composition than traditional solutions used in hybridization applications may comprise a composition with the proviso that the composition does not contain formamide, or with the proviso that the composition contains less than 50%, or less than 25%, or less than 10%, or less than 5%, or less than 2%, or less than 1%, or less than 0.5%, or less than 0.1%, or less than 0.05%, or less than 0.01% formamide. A less-toxic composition may in one disclosure also comprise a composition with the proviso that the composition does not contain dimethyl sulfoxide (DMSO), or with the proviso that the composition contains less than 10%, 5%, 2%, or less than 1%, or less than 0.5%, or less than 0.1%, or less than 0.05%, or less than 0.01% DMSO.

In one embodiment of the invention, suitable polar aprotic solvents for use in the invention may be selected based on their Hansen Solubility Parameters. For example, suitable polar aprotic solvents may have a dispersion solubility parameter between 17.7 to 22.0 $MPa^{1/2}$, a polar solubility parameter between 13 to 23 $MPa^{1/2}$, and a hydrogen bonding solubility parameter between 3 to 13 $MPa^{1/2}$.

According to one embodiment of the present invention, suitable polar aprotic solvents for use in the invention are cyclic compounds. A cyclic compound has a cyclic base structure. Examples include the cyclic compounds disclosed herein. In other embodiments, the polar aprotic solvent may be chosen from Formulas 1-4 below:

Formula 1

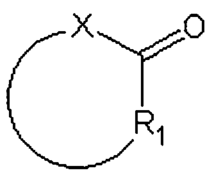

Formula 2

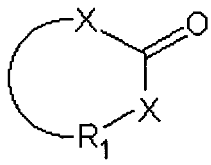

,

Formula 3

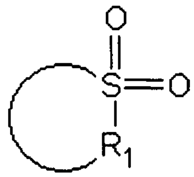

,

Formula 4

, or 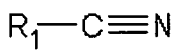

where X is O and R1 is alkyldiyl.

According to another embodiment of the invention, suitable polar aprotic solvents for use in the invention may be chosen from Formula 5 below:

Formula 5

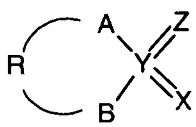

where X is optional and if present, is chosen from O or S;
where Z is optional and if present, is chosen from O or S;
where A and B independently are O or N or S or part of the alkyldiyl or a primary amine;
where R is alkyldiyl; and
where Y is O or S or C.

Examples of suitable polar aprotic solvents according to Formula 5 are provided in Formulas 6-9 below:

Formula 6

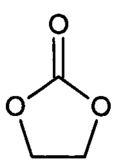

Formula 7

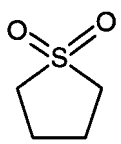

Formula 8

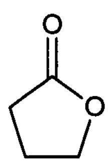

Formula 9

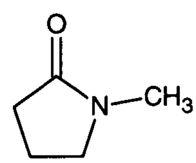

(continued)

| Formula 6 | Formula 7 | Formula 8 | Formula 9 |
|---|---|---|---|
| where:<br>X is non-existing;<br>A, B, and Z are O;<br>Y is C; and<br>R is Ethane-1,2 diyl; | where:<br>Z and X are O;<br>A and B are part of the alkyldiyl;<br>Y is S; and<br>R is Butane-1,4 diyl; | where:<br>X is non-existing;<br>A is part of the alkyldiyl;<br>Y is C;<br>B and Z is O; and<br>R is Propane-1,3 diyl; | where:<br>X is non-existing;<br>A is part of the alkyldiyl;<br>Y is C;<br>B is methylamine;<br>Z is O; and<br>R is Propane-1,3 diyl |

According to yet another embodiment of the invention the polar aprotic solvent has lactone, sulfone, nitrile, sulfite, or carbonate functionality. Such compounds are distinguished by their relatively high dielectric constants, high dipole moments, and solubility in water.

According to another embodiment of the invention the polar aprotic solvent having lactone functionality is γ-butyrolactone (GBL), the polar aprotic solvent having sulfone functionality is sulfolane (SL), the polar aprotic solvent having nitrile functionality is acetonitrile (AN), the polar aprotic solvent having sulfite functionality is glycol sulfite/ethylene sulfite (GS), and the polar aprotic solvent having carbonate functionality is ethylene carbonate (EC), propylene carbonate (PC), or ethylene thiocarbonate (ETC). In yet another aspect of the invention, the compositions and methods of the invention comprise a polar aprotic solvent, with the proviso that the polar aprotic solvent is not acetonitrile (AN) or sulfolane (SL).

In one embodiment, a sufficient amount of energy to hybridize the first and second nucleic acids is provided.

In one embodiment, the first nucleic acid sequence is in a biological sample. In another embodiment, the biological sample is a cytology or histology sample.

In one embodiment, the first nucleic acid sequence is a single stranded sequence and the second nucleic acid sequence is a double stranded sequence. In another embodiment, the first nucleic acid sequence is a double stranded sequence in a biological sample and the second nucleic acid sequence is a single stranded sequence. In yet another embodiment, both the first and second nucleic acid sequences are double stranded. In yet another embodiment, both the first and second nucleic acid sequences are single stranded.

A further aspect of the invention comprises a method wherein the step of providing a sufficient amount of energy to denature the nucleic acids involves a heating step performed by the use of microwaves, hot baths, hot plates, heat wire, peltier element, induction heating, or heat lamps.

According to a further disclosure of the application, the invention relates to the use of a composition comprising between 1 and 95% (v/v) of at least one polar aprotic solvent in hybridization assays.

According to yet another disclosure of the application, the invention relates to the use of a composition comprising an aqueous composition as described herein for use in hybridization assays.

## BRIEF DESCRIPTION OF THE DRAWINGS

FIG. 1 depicts a typical time-course for single locus detection with primary labeled FISH probes on formaldehyde fixed paraffin embedded tissue sections (histological specimens). The bars represent a hybridization assay performed using a traditional solution (top) and a typical time-course for a hybridization assay performed using a composition of the invention (bottom). The first bar on the left in each time-course represents the deparaffination step; the second bar represents the heat-pretreatment step; the third bar represents the digestion step; the fourth bar represents the denaturation and hybridization steps; the fifth bar represents the stringency wash step; and the sixth bar represents the mounting step.

FIG. 2 depicts a typical time-course for single locus detection with primary labeled FISH probes on cytological specimens. The bars represent a hybridization assay performed using a traditional solution (top) and a typical time-course for a hybridization assay performed using a composition of the invention (bottom). The first bar on the left in each time-course represents the fixation step; the second bar represents the denaturation and hybridization steps; the third bar represents the stringency wash step; and the fourth bar represents the mounting step.

## DETAILED DESCRIPTION

### A. Definitions

In the context of the present invention the following terms are to be understood as follows:

"Biological sample" is to be understood as any *in vivo, in vitro,* or *in situ* sample of one or more cells or cell fragments. This can, for example, be a unicellular or multicellular organism, tissue section, cytological sample, chromosome spread, purified nucleic acid sequences, artificially made nucleic acid sequences made by, *e.g.,* a biologic based system or by chemical synthesis, microarray, or other form of nucleic acid chip. In one embodiment, a sample is a mammalian sample, such as, *e.g.,* a human, murine, rat, feline, or canine sample.

"Nucleic acid," "nucleic acid chain," and "nucleic acid sequence" mean anything that binds or hybridizes using base pairing including, oligomers or polymers having a backbone formed from naturally occurring nucleotides and/or nucleic acid analogs comprising nonstandard nucleobases and/or nonstandard backbones *(e.g.,* a peptide nucleic acid (PNA) or locked nucleic acid (LNA)), or any derivatized form of a nucleic acid.

As used herein, the term "peptide nucleic acid" or "PNA" means a synthetic polymer having a polyamide backbone with pendant nucleobases (naturally occurring and modified), including, but not limited to, any of the oligomer or polymer segments referred to or claimed as peptide nucleic acids in, *e.g.,* U.S. Pat. Nos. 5,539,082, 5,527,675, 5,623,049, 5,714,331, 5,718,262, 5,736,336, 5,773,571, 5,766,855, 5,786,461, 5,837,459, 5,891,625, 5,972,610, 5,986,053, 6,107,470 6,201,103, 6,228,982 and 6,357,163, WO96/04000, or any of the references cited therein. The pendant nucleobase, such as, *e.g.,* a purine or pyrimidine base on PNA may be connected to the backbone via a linker such as, *e.g.,* one of the linkers taught in PCT/US02/30573 or any of the references cited therein. In one embodiment, the PNA has an N-(2-aminoethyl)-glycine) backbone. PNAs may be synthesized (and optionally labeled) as taught in PCT/US02/30573 or any of the references cited therein. PNAs hybridize tightly, and witch high sequence specificity, with DNA and RNA, because the PNA backbone is uncharged. Thus, short PNA probes may exhibit comparable specificity to longer DNA or RNA probes. PNA probes may also show greater specificity in binding to complementary DNA or RNA.

As used herein, the term "locked nucleic acid" or "LNA" means an oligomer or polymer comprising at least one or more LNA subunits. As used herein, the term "LNA subunit" means a ribonucleotide containing a methylene bridge that connects the 2'-oxygen of the ribose with the 4'-carbon. See generally, Kurreck, Eur. J. Biochem., 270:1628-44 (2003).

Examples of nucleic acids and nucleic acid analogs also include polymers of nucleotide monomers, including double and single stranded deoxyribonucleotides (DNA), ribonucleotides (RNA), $\alpha$-anomeric forms thereof, synthetic and natural analogs thereof, and the like. The nucleic acid chain may be composed entirely of deoxyribonucleotides, ribonucleotides, peptide nucleic acids (PNA), locked nucleic acids (LNA), synthetic or natural analogs thereof, or mixtures thereof. DNA, RNA, or other nucleic acids as defined herein can be used in the method and compositions of the invention.

"Polar aprotic solvent" refers to an organic solvent having a dipole moment of about 2 debye units or more, a water solubility of at least about 5% (volume) at or near ambient temperature, *i.e.,* about 20°C, and which does not undergo significant hydrogen exchange at approximately neutral pH, *i.e.,* in the range of 5 to 9, or in the range 6 to 8. Polar aprotic solvents include those defined according to the Hansen Solubility Parameters discussed below.

"Alkyldiyl" refers to a saturated or unsaturated, branched, straight chain or cyclic hydrocarbon radical having two monovalent radical centers derived by the removal of one hydrogen atom from each of two different carbon atoms of a parent alkane, alkene, or alkyne.

"Aqueous solution" is to be understood as a solution containing water, even small amounts of water. For example, a solution containing 1% water is to be understood as an aqueous solution.

"Hybridization application," "hybridization assay," "hybridization experiment," "hybridization procedure," "hybridization technique," "hybridization method," etc. are to be understood as referring to any process that involves hybridization of nucleic acids. Unless otherwise specified, the terms "hybridization" and "hybridization step" are to be understood as referring to the re-annealing step of the hybridization procedure as well as the denaturation step (if present).

"Hybridization composition" refers to an aqueous solution of the invention for performing a hybridization procedure, for example, to bind a probe to a nucleic acid sequence. Hybridization compositions may comprise, *e.g.,* at least one polar aprotic solvent, at least one nucleic acid sequence, and a hybridization solution. Hybridization compositions do not comprise enzymes or other components, such as deoxynucleoside triphosphates (dNTPs), for amplifying nucleic acids in a biological sample.

"Hybridization solution" refers to an aqueous solution for use in a hybridization composition of the invention. Hybridization solutions are discussed in detail below and may comprise, e.g., buffering agents, accelerating agents, chelating agents, salts, detergents, and blocking agents.

"PCR composition" refers to an aqueous solution of the invention for performing a hybridization procedure to

amplify a nucleic acid sequence. PCR compositions may comprise, e.g., at least one polar aprotic solvent, at least one enzyme for amplifying nucleic acids, a set of nucleic acid oligonucleotide primers, a mixture of dNTPs, and a PCR solution.

"PCR solution" refers to an aqueous solution for use in a PCR composition of the invention. PCR solutions may comprise e.g., buffering agents, accelerating agents, chelating agents, salts, and detergents.

"Hansen Solubility Parameters" and "HSP" refer to the following cohesion energy (solubility) parameters: (1) the dispersion solubility parameter ($\delta_D$, "D parameter"), which measures nonpolar interactions derived from atomic forces; (2) the polar solubility parameter ($\delta_P$, "P parameter"), which measures permanent dipole-permanent dipole interactions; and (3) the hydrogen bonding solubility parameter ($\delta_H$, "H parameter"), which measures electron exchange. The Hansen Solubility Parameters are further defined below.

"Repetitive Sequences" is to be understood as referring to the rapidly reannealing (approximately 25%) and/or intermediately reannealing (approximately 30%) components of mammalian genomes. The rapidly reannealing components contain small (a few nucleotides long) highly repetitive sequences usually found in tandem *(e.g.,* satellite DNA), while the intermediately reannealing components contain interspersed repetitive DNA. Interspersed repeated sequences are classified as either SINEs (short interspersed repeat sequences) or LINEs (long interspersed repeated sequences), both of which are classified as retrotransposons in primates. SINEs and LINEs include, but are not limited to, Alu-repeats, Kpn-repeats, di-nucleotide repeats, tri-nucleotide repeats, tetra-nucleotide repeats, penta-nucleotide repeats and hexa-nucleotide repeats. Alu repeats make up the majority of human SINEs and are characterized by a consensus sequence of approximately 280 to 300 bp that consist of two similar sequences arranged as a head to tail dimer. In addition to SINEs and LINEs, repeat sequences also exist in chromosome telomeres at the termini of chromosomes and chromosome centromeres, which contain distinct repeat sequences that exist only in the central region of a chromosome. However, unlike SINEs and LINEs, which are dispersed randomly throughout the entire genome, telomere and centromere repeat sequences are localized within a certain region of the chromosome.

"Non-toxic" and "reduced toxicity" are defined with respect to the toxicity labeling of formamide according to "Directive 1999/45/EC of the European Parliament and of the Council of 31 May 1999 concerning the approximation of the laws, regulations and administrative provisions of the Member States relating to the classification, packaging, and labelling of dangerous preparations" (ecb.jrc.it/legislation/1999L0045EC.pdf) ("Directive"). According to the Directive, toxicity is defined using the following classification order: T+ "very toxic"; T "toxic", C "corrosive", Xn "harmful", .Xi "irritant." Risk Phrases ("R phrases") describe the risks of the classified toxicity. Formamide is listed as T (toxic) and R61 (may cause harm to the unborn child). All of the following chemicals are classified as less toxic than formamide: acetonitrile (Xn, R11, R20, R21, R22, R36); sulfolane (Xn, R22); γ-butyrolactone (Xn, R22, R32); and ethylene carbonate (Xi, R36, R37, R38). At the time of filing this application, ethylene trithiocarbonate and glycol sulfite are not presently labeled.

As used herein, the terms "reduced temperature denaturation" and "low temperature denaturation" refer to denaturations performed below about 82°C.

As used herein, the terms "room temperature" and "RT" refer to about 20°C to about 25°C, unless otherwise stated.

**B. Solvent Selection**

Suitable polar aprotic solvents for use in the invention may be selected based on their Hansen Solubility Parameters. Methods for experimentally determining and/or calculating HSP for a solvent are known in the art, and HSP have been reported for over 1200 chemicals.

For example, the D parameter may be calculated with reasonable accuracy based on refractive index, or may be derived from charts by comparison with known solvents of similar size, shape, and composition after establishing a critical temperature and molar volume. The P parameter may be estimated from known dipole moments (*see, e.g.,* McClellan A.L., Tables of Experimental Dipole Moments (W.H. Freeman 1963)) using Equation 1:

$$\text{Equation 1: } \delta_P = 37.4(\text{Dipole Moment})/V^{1/2}$$

where V is the molar volume. There are no equations for calculating the H parameter. Instead, the H parameter is usually determined based on group contributions.

HSP characterizations are conveniently visualized using a spherical representation, with the HSP of an experimentally-determined suitable reference solvent at the center of the sphere. The radius of the sphere (R) indicates the maximum tolerable variation from the HSP of the reference solvent that still allows for a "good" interaction to take place. Good solvents are within the sphere and bad ones are outside. The distance, $R_a$, between two solvents based on their respective HSP values can be determined using Equation 2:

$$\text{Equation 2: } (R_a)^2 = 4(\delta_{D1} - \delta_{D2})^2 + (\delta_{P1} - \delta_{P2})^2 (\delta_{H1} - \delta_{H2})^2$$

where subscript 1 indicates the reference sample, subscript 2 indicates the test chemical, and all values are in $MPa^{1/2}$. Good solubility requires that $R_a$ be less than the experimentally-determined radius of the solubility sphere $R_o$. The relative energy difference between two solvents, *i.e.,* RED number, can be calculated by taking the ratio of $R_a$ to $R_o$, as shown in Equation 3.

$$\text{Equation 3: RED} = R_a/R_o$$

RED numbers less than 1.0 indicate high affinity; RED numbers equal or close to 1.0 indicate boundary conditions; and progressively higher RED numbers indicate progressively lower affinities.

In some embodiments, the D parameters of the polar aprotic solvents of the invention are between 17.7 to 22.0 $MPa^{1/2}$. Such relatively high D parameters are generally associated with solvents having cyclic structures and/or structures with sulfur or halogens. Linear compounds are not likely to be among the most suitable polar aprotic solvents for use in the invention, but may be considered if their P and H parameters are within the ranges discussed below. Since the D parameter is multiplied by 4 in Equation 2, the limits are one-half of $R_o$. In addition, it should be noted that D values of around 21 or higher are often characteristic of a solid.

In some embodiments, the P parameters of the polar aprotic solvents of the invention are between 13 to 23 $MPa^{1/2}$. Such exceptionally high P parameters are generally associated with solvents having a high dipole moment and presumably also a relatively low molecular volume. For example, for V near 60 cc/mole, the dipole moment should be between 4.5 and 3.1. For V near 90 cc/mole, the dipole moment should be between 5.6 and 3.9.

In some embodiments, the H parameters of the polar aprotic solvents of the invention are between 3 to 13 $MPa^{1/2}$ . Generally, polar aprotic solvents having an alcohol group are not useful in the compositions and methods of the invention, since the H parameters of such solvents would be too high.

The molar volume of the polar aprotic solvent may also be relevant, since it enters into the evaluation of all three Hansen Solubility Parameters. As molar volume gets smaller, liquids tend to evaporate rapidly. As molar volume gets larger, liquids tend to enter the solid region in the range of D and P parameters recited above. Thus, the polar aprotic solvents of the invention are rather close to the liquid/solid boundary in HSP space.

In some embodiments, the polar aprotic solvents of the invention have lactone, sulfone, nitrile, sulfite, and/or carbonate functionality. Such compounds are distinguished by their relatively high dielectric constants, high dipole moments, and solubility in water. An exemplary polar aprotic solvent with lactone functionality is γ-butyrolactone (GBL), an exemplary polar aprotic solvent with sulfone functionality is sulfolane (SL; tetramethylene sulfide-dioxide), an exemplary polar aprotic solvent with nitrile functionality is acetonitrile (AN), an exemplary polar aprotic solvent with sulfite functionality is glycol sulfite/ethylene sulfite (GS), and an exemplary polar aprotic solvents with carbonate functionality are ethylene carbonate (EC), propylene carbonate (PC), or ethylene trithiocarbonate (ETC). The structures of these exemplary solvents are provided below and their Hansen Solubility Parameters, RED numbers, and molar volumes are given in Table 1.

| | | | | | |
|---|---|---|---|---|---|
| 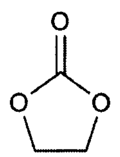 | 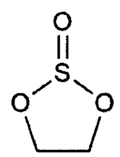 | 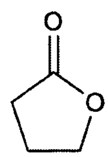 | 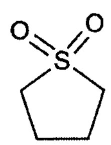 | 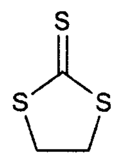 | 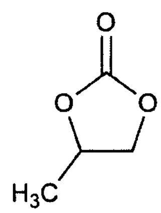 |
| ethylene carbonate | glycol sulfite | γ-butyrolactone | sulfolane | ethylene trithiocarbonate | propylene carbonate |

Table 1

| | D | P | H | RED | Molar Volume ($cm^3$/mole) |
|---|---|---|---|---|---|
| Correlation ($R_o$ = 3.9) | 19.57 | 19.11 | 7.71 | - | - |
| GBL | 19.0 | 16.6 | 7.4 | 0.712 | 76.5 |

(continued)

| | D | P | H | RED | Molar Volume ($cm^3$/mole) |
|---|---|---|---|---|---|
| PC | 20.0 | 18.0 | 4.1 | 0.993 | 85.2 |
| SL | 20.3 | 18.2 | 10.9 | 0.929 | 95.7 |
| EC | 19.4 | 21.7 | 5.1 | 0.946 | 66.0 |
| ETC | n/a | n/a | n/a | n/a | n/a |
| GS | 20.0 | 15.9 | 5.1 | n/a | 75.1 |
| n/a = not available. | | | | | |

Other suitable polar aprotic solvents that may be used in the invention are cyclic compounds such as, *e.g.*, ε-caprolactone. In addition, substituted pyrolidinones and related structures with nitrogen in a 5- or 6-membered ring, and cyclic structures with two nitrile groups, or one bromine and one nitrile group, may also be suitable for use in the invention. For example, N-methyl pyrrolidinone (shown below) may be a suitable polar aprotic solvent for use in the methods and compositions of the invention.

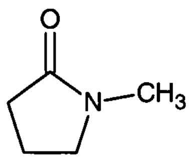

Other suitable polar aprotic solvents may contain a ring urethane group (NHCOO-). However, not all such compounds are suitable, since 1,3-dimethyl-2-imidazolidinone produces no signals when used in the hybridization compositions of the invention. One of skill in the art may screen for compounds useful in the compositions and methods of the invention as described herein. Exemplary chemicals that may be suitable for use in the invention are set forth in Tables 2 and 3 below.

Table 2

| Solvent | D | P | H |
|---|---|---|---|
| Acetanilide | 20.6 | 13.3 | 12.4 |
| N-Acetyl Pyrrolidone | 17.8 | 13.1 | 8.3 |
| 4-Amino Pyridine | 20.4 | 16.1 | 12.9 |
| Benzamide | 21.2 | 14.7 | 11.2 |
| Benzimidazole | 20.6 | 14.9 | 11.0 |
| 1,2,3-Benzotriazole | 18.7 | 15.6 | 12.4 |
| Butadienedioxide | 18.3 | 14.4 | 6.2 |
| 2,3-Butylene Carbonate | 18.0 | 16.8 | 3.1 |
| Caprolactone (Epsilon) | 19.7 | 15.0 | 7.4 |
| Chloro Maleic Anhydride | 20.4 | 17.3 | 11.5 |
| 2-Chlorocyclohexanone | 18.5 | 13.0 | 5.1 |
| Chloronitromethane | 17.4 | 13.5 | 5.5 |
| Citraconic Anhydride | 19.2 | 17.0 | 11.2 |
| Crotonlactone | 19.0 | 19.8 | 9.6 |
| Cyclopentanone | 17.8 | 11.9 | 5.2 |
| Cyclopropylnitrile | 18.6 | 16.2 | 5.7 |
| Dimethyl Sulfate | 17.7 | 17.0 | 9.7 |

(continued)

| Solvent | D | P | H |
|---|---|---|---|
| Dimethyl Sulfone | 19.0 | 19.4 | 12.3 |
| | | | |
| 1,2-Dinitrobenzene | 20.6 | 22.7 | 5.4 |
| 2,4-Dinitrotoluene | 20.0 | 13.1 | 4.9 |
| Dipheynyl Sulfone | 21.1 | 14.4 | 3.4 |
| 1,2-Dinitrobenzene | 20.6 | 22.7 | 5.4 |
| 2,4-Dinitrotoluene | 20.0 | 13.1 | 4.9 |
| Epsilon-Caprolactam | 19.4 | 13.8 | 3.9 |
| Ethanesulfonylchloride | 17.7 | 14.9 | 6.8 |
| N-Ethyl-2-Pyrrolidone | 18.0 | 12.0 | 7.0 |
| N-Formyl Piperidine | 18.7 | 10.6 | 7.8 |
| Furfural | 18.6 | 14.9 | 5.1 |
| 2-Furonitrile | 18.4 | 15.0 | 8.2 |
| Isoxazole | 18.8 | 13.4 | 11.2 |
| Maleic Anhydride | 20.2 | 18.1 | 12.6 |
| Malononitrile | 17.7 | 18.4 | 6.7 |
| 4-Methoxy Benzonitrile | 19.4 | 16.7 | 5.4 |
| 1-Methoxy-2-Nitrobenzene | 19.6 | 16.3 | 5.5 |
| 1-Methyl Imidazole | 19.7 | 15.6 | 11.2 |
| 3-Methyl Isoxazole | 19.4 | 14.8 | 11.8 |
| N-Methyl-2-Pyrrolidone | 18.0 | 12.3 | 7.2 |
| N-Methyl Morpholine-N-Oxide | 19.0 | 16.1 | 10.2 |
| Methyl Phenyl Sulfone | 20.0 | 16.9 | 7.8 |
| Methyl Sulfolane | 19.4 | 17.4 | 5.3 |
| Methyl-4-Toluenesulfonate | 19.6 | 15.3 | 3.8 |
| 3-Nitroaniline | 21.2 | 18.7 | 10.3 |
| 2-Nitrothiophene | 19.7 | 16.2 | 8.2 |
| 9,10-Phenanthrenequinone | 20.3 | 17.1 | 4.8 |
| Phthalic Anhydride | 20.6 | 20.1 | 10.1 |
| 1,3-Propane Sultone | 18.4 | 16.0 | 9.0 |
| beta-Propiolactone | 19.7 | 18.2 | 10.3 |
| 2-Pyrrolidone | 19.4 | 17.4 | 11.3 |
| Saccharin | 21.0 | 13.9 | 8.8 |
| Succinonitrile | 17.9 | 16.2 | 7.9 |
| Sulfanilamide | 20.0 | 19.5 | 10.7 |
| Sulfolane | 20.3 | 18.2 | 10.9 |
| 2,2,6,6-Tetrachlorocyclohexanone | 19.5 | 14.0 | 6.3 |
| Tetramethylene Sulfoxide | 18.2 | 11.0 | 9.1 |

(continued)

| Solvent | D | P | H |
|---|---|---|---|
| Thiazole | 20.5 | 18.8 | 10.8 |
| 3,3,3-Trichloro Propene | 17.7 | 15.5 | 3.4 |
| 1,1,2-Trichloro Propene | 17.7 | 15.7 | 3.4 |
| 1,2,3-Trichloro Propene | 17.8 | 15.7 | 3.4 |
| Vinylene carbonate | 17.3 | 18.1 | 9.6 |

Table 2 sets forth an exemplary list of potential chemicals for use in the compositions and methods of the invention based on their Hansen Solubility Parameters. Other compounds, may of course, also meet these requirements such as, for example, those set forth in Table 3.

Table 3

| Chemical (dipole moment) | RED | Melting Point °C |
|---|---|---|
| Chloroethylene carbonate (4.02) | 0.92 | - |
| 2-Oxazolidinone (5.07) | 0.48 | 86-89 |
| 2-Imidazole | 1.49 | 90-91 |
| 1,5-Dimethyl Tetrazole (5.3) | ~1.5 | 70-72 |
| N-Ethyl Tetrazole (5.46) | -1.5 | |
| Trimethylene sulfide-dioxide (4.49) | - | - |
| Trimethylene sulfite (3.63) | - | - |
| 1,3-Dimethyl-5-Tetrazole (4.02) | - | - |
| Pyridazine (3.97) | 1.16 | -8 |
| 2-Thiouracil (4.21) | - | - |
| N-Methyl Imidazole (6.2) | 1.28 | - |
| 1-Nitroso-2-pyrolidinone | -1.37 | - |
| Ethyl Ethyl Phosphinate (3.51) | - | - |
| 5-cyano-2-Thiouracil (5.19) | - | - |
| 4H-Pyran-4-thione (4.08) | 1.35 | 32-34 |
| 4H-Pyran-4-one = gamma pyrone (4.08) | 1.49 | Boiling Point (BP) 80 |
| 2-Nitrofuran (4.41) | 1.14 | 29 |
| Methyl alpha Bromo Tetronate (6.24) | - | - |
| Tetrahydrothiapyran oxide (4.19) | 1.75 | 60-64 |
| Picolinonitrile (2-cyanopyridine) (5.23) | 0.40 | 26-28 (BP 212-215) |
| Nitrobenzimidazole (6.0) | 0.52 | 207-209 |
| Isatin (5.76) | - | 193-195 |
| N-phenyl sydnone (6.55) | - | - |
| Glycol sulfate (Ethylene glycol) Note: not soluble at 40% | - | 99°C |

Some of the chemicals listed in Tables 2 and 3 have been used in hybridization and/or PCR applications in the prior art (*e.g.*, dimethyl sulfoxide (DMSO) has been used in hybridization and PCR applications, and sulfolane (SL), acetonitrile (AN), 2-pyrrolidone, ε-caprolactam, and ethylene glycol have been used in PCR applications). As stated above, the polar aprotic solvent in the invention is not DMSO. In some embodiments, the polar aprotic solvent is not

sulfolane, acetonitrile, 2-pyrrolidone, ε-caprolactam, or ethylene glycol. However, most polar aprotic solvents have not been used in prior art hybridization applications. Moreover, even when such compounds were used, the prior art did not recognize that they may be advantageously used to decrease denaturation temperatures or eliminate the denaturation step from hybridization applications, as disclosed in this application.

In addition, not all of the chemicals listed in Tables 2 and 3 are suitable for use in the compositions and methods of the invention. For example, although DMSO's Hansen Solubility Parameters (HSPs) fall within the ranges recited above, DMSO does not function to eliminate the denauturation step in the methods of the invention. However, it is well within the skill of the ordinary artisan to screen for suitable compounds using the guidance provided herein including testing a compound in one of the examples provided. For example, in some embodiments, suitable polar aprotic solvents will have HSPs within the ranges recited above and a structure shown in Formulas 1-9 above.

Additional exemplary polar aprotic solvents suitable for use in the methods of the invention include delta-valerolactone (2-piperidone), gamma-valerolactone, sulfolene (butadiene sulfone), pentamethylene sulfone, and 1,2-dioxan-2-one.

In some embodiments, the polar aprotic solvent is chosen from ethylene carbonate, sulfolane, gamma-butyrolactone, and propylene carbonate. In other embodiments, the polar aprotic solvent is chosen from ethylene carbonate, sulfolane, gamma-butyrolactone, propylene carbonate, ethylene trithiocarbonate, glycol sulfite/ethylene sulfite, delta-valerolactam (2-piperidone), and tetrahydrothiophene 1-oxide. In yet other embodiments, the polar aprotic solvent is chosen from ethylene carbonate, sulfolane, gamma-butyrolactone, propylene carbonate, ethylene trithiocarbonate, glycol sulfite/ethylene sulfite, delta-valerolactam (2-piperidone), 2-pyrrolidone, tetrahydrothiophene 1-oxide, pentamethylene sulfone, and 1,2-dioxan-2-one.

### C. Compositions, Buffers, and Solutions

#### (1) Hybridization Solutions

Traditional hybridization solutions are known in the art. Such solutions may comprise, for example, buffering agents, accelerating agents, chelating agents, salts, detergents, and blocking agents.

For example, the buffering agents may include SSC, HEPES, SSPE, PIPES, TMAC, TRIS, SET, citric acid, a phosphate buffer, such as, e.g., potassium phosphate or sodium pyrrophosphate, etc. The buffering agents may be present at concentrations from 0.01 x to 50x, such as, for example, 0.01x, 0.1x, 0.5x, 1x, 2x, 5x, 10x, 15x, 20x, 25x, 30x, 35x, 40x, 45x, or 50x. Typically, the buffering agents are present at concentrations from 0.1x to 10x.

The accelerating agents may include polymers such as FICOLL, PVP, heparin, dextran sulfate, proteins such as BSA, glycols such as ethylene glycol, glycerol, 1,3 propanediol, propylene glycol, or diethylene glycol, combinations thereof such as Dernhardt's solution and BLOTTO, and organic solvents such as formamide, dimethylformamide, etc. The accelerating agent may be present at concentrations from 1% to 80% or 0.1x to 10x, such as, for example, 0.1% (or 0.1x), 0.2% (or 0.2x), 0.5% (or 0.5x), 1% (or 1x), 2% (or 2x), 5% (or 5x), 10% (or 10x), 15% (or 15x), 20% (or 20x), 25% (or 25x), 30% (or 30x), 40% (or 40x), 50% (or 50x), 60% (or 60x), 70% (or 70x), or 80% (or 80x). Typically, formamide is present at concentrations from 25% to 75%, such as 25%, 30%, 40%, 50%, 60%, 70%, or 75% , while dextran sulfate, and glycol are present at concentrations from 5% to 10%, such as 5%, 6%, 7%, 8%, 9%, or 10%.

The chelating agents may include EDTA, EGTA, etc. The chelating agents may be present at concentrations from 0.1 mM to 10 mM, such as 0.1mM, 0.2mM, 0.5mM, 1mM, 2mM, 3mM, 4mM, 5mM, 6mM, 7mM, 8mM, 9mM, or 10mM. Typically, the chelating agents are present at concentrations from 0.5 mM to 5 mM, such as 0.5mM, 1mM, 1.5mM, 2mM, 2.5mM, 3mM, 3.5mM, 4mM. 4.5mM, or 5mM.

The salts may include sodium chloride, sodium phosphate, magnesium phosphate, etc. The salts may be present at concentrations from 1 mM to 750 mM, such as 1mM, 5mM, 10mM, 20mM, 30mM, 40mM, 50mM, 100mM, 200mM, 300mM, 400mM, 500mM, 600mM, 700mM, or 750mM. Typically, the salts are present at concentrations from 10 mM to 500 mM, such as 10mM, 20mM, 30mM, 40mM, 50mM, 100mM, 200mM, 300mM, 400mM, or 500mM.

The detergents may include Tween, SDS, Triton, CHAPS, deoxycholic acid, etc. The detergent may be present at concentrations from 0.001% to 10%, such as, for example, 0.0001, 0.01, 0.1, 0.5, 1, 2, 3, 4, 5, 6, 7, 8, 9, or 10%. Typically, the detergents are present at concentrations from 0.01% to 1%, such as 0.01%, 0.02%, 0.03%, 0.05%, 0.1%, 0.2%, 0.3%, 0.4%, 0.5%, 0.6%, 0.7%, 0.8%, 0.9%, or 1%.

The nucleic acid blocking agents may include, yeast tRNA, homopolymer DNA, denatured salmon sperm DNA, herring sperm DNA, total human DNA, COT1 DNA, etc. The blocking nucleic acids may be present at concentrations of 0.05 mg/mL to 100 mg/mL. However, the compositions and methods of the invention surprisingly show significantly reduced background levels without the need for blocking agents, and without the need for overnight hybridization in formamide-containing buffers.

A great variation exists in the literature regarding traditional hybridization solutions. For example, a traditional hybridization solution may comprise 5x or 6x SSC, 0.01 M EDTA, 5x Dernhardt's solution, 0.5% SDS, and 100 mg/mL

sheared, denatured salmon sperm DNA. Another traditional hybridization solution may comprise 50 mM HEPES, 0.5 M NaCl, and 0.2 mM EDTA. A typical hybridization solution for FISH on biological specimens for RNA detection may comprise, *e.g.,* 2x SSC, 10% dextran sulfate, 2 mM vanadyl-ribonucleoside complex, 50% formamide, 0.02% RNAse-free BSA, and 1 mg/mL *E. coli* tRNA. A typical hybridization solution for FISH on biological specimens for DNA detection may comprise, e.g., 2x SSC, 10% dextran sulfate, 50% formamide, and *e.g.,* 0.3 mg/mL salmon sperm DNA or 0.1 mg/mL COT1 DNA. Other typical hybridization solutions may comprise 40% formamide, 10% dextran sulfate, 300 mM NaCl, 5 mM phosphate buffer, Alu-PNA (blocking PNA) or COT-1 DNA, and in some cases 0.1 μg/μL total human DNA (THD).

The compositions of the invention may comprise a hybridization solution comprising any of the components of traditional hybridization solutions recited above in combination with at least one polar aprotic solvent. The traditional components may be present at the same concentrations as used in traditional hybridization solutions, or may be present at higher or lower concentrations, or may be omitted completely.

For example, if the compositions of the invention comprise salts such as NaCl and/or phosphate buffer, the salts may be present at concentrations of 0-1200 mM NaCl and/or 0-200 mM phosphate buffer. In some embodiments, the concentrations of salts may be, for example, 300 mM NaCl and/or 5 mM phosphate buffer, or 600 mM NaCl and/or 10 mM phosphate buffer.

If the compositions of the invention comprise accelerating agents such as dextran sulfate or glycol, the dextran sulfate may be present at concentrations of from 5% to 40% and the glycol may be present at concentrations of from 0.1% to 10%. In some embodiments, the concentration of dextran sulfate may be 10% or 20% and the concentration of ethylene glycol, 1,3-propanediol, or glycerol may be 1% to 10%.. In some embodiments, the aqueous composition does not comprise formamide as an accelerating agent, or comprises formamide with the proviso that the composition contains less than 50%, or less than 25%, or less than 10%, or less than 5%, or less than 2%, or less than 1%, or less than 0.5%, or less than 0.1%, or less than 0.05%, or less than 0.01%.

If the compositions of the invention comprise citric acid, the concentrations may range from 1 mM to 50 mM and the pH may range from 5.0 to 8.0. In some embodiments the concentration of citric acid may be 10 mM and the pH may be 6.2.

The compositions of the invention may comprise agents that reduce non-specific binding to, for example, the cell membrane, such as salmon sperm or small amounts of total human DNA or, for example, they may comprise blocking agents to block binding of, *e.g.,* repeat sequences to the target such as larger amounts of total human DNA or repeat enriched DNA or specific blocking agents such as PNA or LNA fragments and sequences. These agents may be present at concentrations of from 0.01-100 μg/μL or 0.01-100 μM. For example, in some embodiments, these agents will be 0.1 1 μg/μL total human DNA, or 0.1 μg/μL non-human DNA, such as herring sperm, salmon sperm, or calf thymus DNA, or 5 μM blocking PNA. However, the compositions and methods of the invention surprisingly show significantly reduced background levels without the need for blocking agents, and without the need for overnight hybridization in formamide-containing buffers.

One aspect of the invention is a composition or solution for use in hybridization. Compositions for use in the invention include an aqueous composition comprising a nucleic acid sequence and at least one polar aprotic solvent in an amount effective to denature double-stranded nucleotide sequences. An amount effective to denature double-stranded nucleotide sequences is an amount that enables hybridization. For example, one way to test for whether the amount of polar aprotic solvent is effective to enable hybridization is to determine whether the polar aprotic solvent, when used in the hybridization methods and compositions described herein, such as example 1, yield a detectable signal and/or an amplified nucleic acid product.

Non-limiting examples of effective amounts of polar aprotic solvents include, e.g., about 1% to about 95% (v/v). In some embodiments, the concentration of polar aprotic solvent is 5% to 60% (v/v). In other embodiments, the concentration of polar aprotic solvent is 10% to 60% (v/v). In still other embodiments, the concentration of polar aprotic solvent is 30% to 50% (v/v). Concentrations of 1% to 5%, 5% to 10%, 10%, 10% to 20%, 20% to 30%, 30% to 40%, 40% to 50%, or 50% to 60% (v/v) are also suitable. In some embodiments, the polar aprotic solvent will be present at a concentration of 0.1%, 0.25%, 0.5%, 1%, 2%, 3%, 4%, or 5% (v/v). In other embodiments, the polar aprotic solvent will be present at a concentration of 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, or 20% (v/v).

If the disclosed compositions are used in a hybridization assay, they may further comprise one or more nucleic acid probes. The probes may be directly or indirectly labeled with detectable compounds such as enzymes, chromophores, fluorochromes, and haptens. The DNA probes may be present at concentrations of 0.1 to 100 ng/μL. For example, in some embodiments, the probes may be present at concentrations of 1 to 10 ng/μL. The PNA probes may be present at concentrations of 0.5 to 5000 nM. For example, in some embodiments, the probes may be present at concentrations of 5 to 1000 nM.

In one embodiment, a disclosed composition comprises a mixture of 40% polar aprotic solvent (v/v) (*e.g.*, ethylene carbonate, "EC"), 10% dextran sulfate, 300 mM NaCl, 5 mM phosphate buffer, and 1-10 ng/μL probe. Another

exemplary composition comprises a mixture of 15% EC, 20% dextran sulfate, 600 mM NaCl, 10 mM phosphate buffer, and 0.1 μg/μl total human DNA. Yet another exemplary composition comprises 15% EC, 20% dextran sulfate, 600 mM NaCl, 10 mM citric acid pH 6.2, and 0.1 μg/μL non-human DNA (*e.g.,* herring sperm, salmon sperm, or calf thymus) OR 0.5% formamide OR 1% glycol (*e.g.,* ethylene glycol, 1,3 propanediol, or glycerol). A further exemplary composition comprises 15% EC, 20% dextran sulfate, 600 mM NaCl, 10 mM citrate buffer pH 6.2.

(2) Polar Aprotic Solvent(s)

Different polar aprotic solvents may impart different properties on the disclosed compositions. For example, the choice of polar aprotic solvent may contribute to the stability of the composition, since certain polar aprotic solvents may degrade over time. For example, the polar aprotic solvent ethylene carbonate breaks down into ethylene glycol, which is a relatively stable molecule, and carbon dioxide, which can interact with water to form carbonic acid, altering the acidity of the compositions of the invention. Without being bound by theory, it is believed that the change in pH upon breakdown of ethylene carbonate and DNA damage from long storage makes the compositions of the invention less effective for hybridization. However, stability can be improved by reducing the pH of the composition, by adding citric acid as a buffer at pH 6.2 instead of the traditional phosphate buffer, which is typically used at about pH 7.4, and/or by adding ethylene glycol at concentrations, e.g., between 0.1% to 10%, or between 0.5% to 5%, such as, for example, 1%, 2%, 3%, etc. For example, with 10 mM citrate buffer, the disclosed compositions are stable at 2-8°C for approximately 8 months. Stability can also be improved if the compositions are stored at low temperatures (*e.g.,* -20°C).

In addition, certain polar aprotic solvents may cause the disclosed compositions to separate into multi-phase systems under certain conditions. The conditions under which multi-phase systems are obtained may be different for different polar aprotic solvents. Generally, however, as the concentration of polar aprotic solvent increases, the number of phases increases. For example, compositions comprising low concentrations ethylene carbonate *(i.e.,* less than 20%) may exist as one phase, while compositions comprising higher concentrations of ethylene carbonate may separate into two, or even three phases. For instance, compositions comprising 15% ethylene carbonate exist as a single phase at room temperature, while compositions comprising 40% ethylene carbonate consist of a viscous lower phase (approximately 25% of the total volume) and a less viscous upper phase (approximately 75% of the total volume) at room temperature.

On the other hand, some polar aprotic solvents may exist in two phases at room temperature even at low concentrations. For example, sulfolane, γ-butyrolactone, ethylene trithiocarbonate, glycol sulfite, and propylene carbonate exist as two phases at concentrations of 10, 15, 20, or 25% (20% dextran sulfate, 600 mM NaCl, 10 mM citrate buffer) at room temperature.

It may also be possible to alter the number of phases by adjusting the temperature of the disclosed compositions. Generally, as temperature increases, the number of phases decreases. For example, at 2-8°C, compositions comprising 40% ethylene carbonate may separate into a three-phase system.

It may also be possible to alter the number of phases by adjusting the concentration of dextran sulfate and/or salt in the composition. Generally speaking, lowering the dextran sulfate concentration (traditional concentration is 10%) and/or salt concentration may reduce the number of phases. However, depending on the particular polar aprotic solvent and its concentration in the composition, single phases may be produced even with higher concentrations of salt and dextran sulfate. For example, a composition comprising low amounts of EC *(e.g.,* 15%, 10%, or 5%) can work well by increasing the dextran sulfate and salt concentrations, while still keeping a one phase system. In a particular embodiment, compositions comprising a HER2 gene DNA probe, a CEN7 PNA probe, 15% EC, 20% dextran sulfate, 600 mM NaCl, and 10 mM phosphate buffer are frozen at -20°C. In other embodiments, the compositions are liquid at -20°C.

Some polar aprotic solvents may produce stronger signals in one phase or another. For example, 40% glycol sulfite produces strong signals in the lower phase and no signals in the upper phase. Similarly, certain types of probes may produce stronger signals in one phase or another. For example, PNA probes tend to show stronger signals in the lower phase than the upper phase.

Accordingly, the disclosed multiphase systems may be used to conveniently examine different aspects of a sample. For example, a two-phase system could be used to separate samples labeled with PNA probes from samples labeled with DNA probes. Other uses include isolation of a specific phase exhibiting, *e.g*., certain advantages such that the isolated phase can be used as a single phase system. The probe and/or sample may be added prior to, or after isolation of a particular phase.

Hybridization applications may be performed with a disclosed one-phase composition, with individual phases of the disclosed multiphase compositions, or with mixtures of any one or more of the phases in a disclosed multiphase composition. For example, in a one phase system, a volume of the sample may be extracted for use in the hybridization. In a multiphase system, one may extract a volume of sample from the phase of interest (*e.g*., the upper, lower, or middle phase) to use in the hybridization. Alternatively, the phases in a multiphase system may be mixed prior to extracting a volume of the mixed sample for use in the hybridization. However, the multiphase system may yield strong and uneven

local background staining depending on the composition. While, the addition of low amounts of formamide will reduce background in a one phase system, it has little effect on a multiphase system with high concentrations *(e.g.,* 40%) of a polar aprotic solvent. In addition, as the concentration of formamide increases, higher concentrations of probe and/or longer hybridization times are required to maintain strong signal intensity.

(3) Optimization for Particular Applications

The disclosed compositions can be varied in order to optimize results for a particular application. For example, the concentration of polar aprotic solvent, salt, accelerating agent, blocking agent, and/or hydrogen ions *(i.e.* pH) may be varied in order to improve results for a particular application.

For example, the concentration of polar aprotic solvent may be varied in order to improve signal intensity and background staining. Generally, as the concentration of polar aprotic solvent increases, signal intensity increases and background staining decreases. For example, compositions comprising 15% EC tend to show stronger signals and less background than compositions comprising 5% EC. However, signal intensity may be improved for compositions having low concentrations of polar aprotic solvent (*e.g.*, 0% to 20%) if the concentrations of salt and/or dextran sulfate are increased. For example, strong signals may be observed with 5% to 10% EC when the salt concentration is raised approximately 3 to 4 times traditional salt concentrations (*i.e.,* approximately 1200 mM NaCl, 20 mM phosphate buffer; traditional salt concentrations are about 300mM NaCl). Likewise, as lower concentrations of polar aprotic solvent are used, higher concentrations of dextran sulfate are generally required to maintain good signal and background intensity.

Accordingly, the concentrations of salt and dextran sulfate may also be varied in order to improve signal intensity and background staining. Generally, as the concentrations of salt and dextran sulfate increase, the signal intensity increases and background decreases. For example, salt concentrations that are approximately two to four times traditional concentrations (*i.e.,* 300 mM NaCl 5 mM phosphate buffer) produce strong signals and low background. Surprisingly, however, hybridization occurs using the compositions of the invention even in the complete absence of salt. Signal intensities can be improved under no-salt conditions by increasing the concentrations of accelerating agent and/or polar aprotic solvent.

Likewise, signal intensity increases as dextran sulfate concentration increases from 0% to 20%. However, good signals may even be observed at dextran sulfate concentrations of 0%. Signal intensity may be improved under low dextran sulfate conditions by increasing the polar aprotic solvent and/or salt concentrations.

In addition, the types probes used in the disclosed compositions may be varied to improve results. For example, in some aspects of the invention, combinations of DNA/DNA probes may show less background than combinations of DNA/PNA probes in the disclosed compositions or vice versa. On the other hand, PNA probes tend to show stronger signals than DNA probes under low salt and/or low polar aprotic solvent concentrations. In fact, PNA probes also show signals when no polar aprotic solvent is present, whereas DNA probes show weak or no signals without polar aprotic solvent.

**D. Applications, Methods, and Uses**

**(1) Analytical Samples**

The methods of the invention may be used fully or partly in all types of hybridization applications in the fields of cytology, histology, or molecular biology. According to one embodiment, the first or the second nucleic acid sequence in the methods of the invention is present in a biological sample. Examples of such samples include, *e.g.*, tissue samples, cell preparations, cell fragment preparations, and isolated or enriched cell component preparations. The sample may originate from various tissues such as, *e.g.,* breast, lung, colorectal, prostate, lung, head & neck, stomach, pancreas, esophagus, liver, and bladder, or other relevant tissues and neoplasia thereof, any cell suspension, blood sample, fine needle aspiration, ascites fluid, sputum, peritoneum wash, lung wash, urine, feces, cell scrape, cell smear, cytospin or cytoprep cells.

The sample may be isolated and processed using standard protocols. Cell fragment preparations may, *e.g.,* be obtained by cell homogenizing, freeze-thaw treatment or cell lysing. The isolated sample may be treated in many different ways depending of the purpose of obtaining the sample and depending on the routine at the site. Often the sample is treated with various reagents to preserve the tissue for later sample analysis, alternatively the sample may be analyzed directly. Examples of widely used methods for preserving samples are formalin-fixed followed by paraffin-embedding and cryo-preservation.

For metaphase spreads, cell cultures are generally treated with colcemid, or another suitable spindle pole disrupting agent, to stop the cell cycle in metaphase. The cells are then fixed and spotted onto microscope slides, treated with formaldehyde, washed, and dehydrated in ethanol. Probes are then added and the samples are analyzed by any of the techniques discussed below.

Cytology involves the examination of individual cells and/or chromosome spreads from a biological sample. Cytological examination of a sample begins with obtaining a specimen of cells, which can typically be done by scraping, swabbing or brushing an area, as in the case of cervical specimens, or by collecting body fluids, such as those obtained from the chest cavity, bladder, or spinal column, or by fine needle aspiration or fine needle biopsy, as in the case of internal tumors. In a conventional manual cytological preparation, the sample is transferred to a liquid suspending material and the cells in the fluid are then transferred directly or by centrifugation-based processing steps onto a glass microscope slide for viewing. In a typical automated cytological preparation, a filter assembly is placed in the liquid suspension and the filter assembly both disperses the cells and captures the cells on the filter. The filter is then removed and placed in contact with a microscope slide. The cells are then fixed on the microscope slide before analysis by any of the techniques discussed below.

In a traditional DNA hybridization experiment using a cytological sample, slides containing the specimen are immersed in a formaldehyde buffer, washed, and then dehydrated in ethanol. The probes are then added and the specimen is covered with a coverslip. The slide is optionally incubated at a temperature sufficient to denature any double-stranded nucleic acid in the specimen (*e.g*., 5 minutes at 82°C) and then incubated at a temperature sufficient to allow hybridization (*e.g*., overnight at 45°C). After hybridization, the coverslips are removed and the specimens are subjected to a high-stringency wash (*e.g.,* 10 minutes at 65°C) followed by a series of low-stringency washes (*e.g.,* 2 x 3 minutes at room temperature). The samples are then dehydrated and mounted for analysis.

In a traditional RNA hybridization experiment using cytological samples, cells are equilibrated in 40% formamide, 1x SSC, and 10 mM sodium phosphate for 5 min, incubated at 37° C overnight in hybridization reactions containing 20 ng of oligonucleotide probe (e.g mix of labeled 50 bp oligos), 1xSSC, 40% formamide, 10% dextran sulfate, 0.4% BSA, 20 mM ribonucleotide vanadyl complex, salmon testes DNA (10 mg/ml), *E. coli* tRNA (10 mg/ml), and 10 mM sodium phosphate. Then washed twice with 4xSSC/40% formamide and again twice with 2x SSC/40% formamide, both at 37° C, and then with 2x SSC three times at room temperature. Digoxigenin-labeled probes can then e.g. be detected by using a monoclonal antibody to digoxigenin conjugated to Cy3. Biotin-labeled probes can then e.g. be detected by using streptavidin-Cy5. Detection can be by fluorescence or CISH.

Histology involves the examination of cells in thin slices of tissue. To prepare a tissue sample for histological examination, pieces of the tissue are fixed in a suitable fixative, typically an aldehyde such as formaldehyde or glutaraldehyde, and then embedded in melted paraffin wax. The wax block containing the tissue sample is then cut on a microtome to yield thin slices of paraffin containing the tissue, typically from 2 to 10 microns thick. The specimen slice is then applied to a microscope slide, air dried, and heated to cause the specimen to adhere to the glass slide. Residual paraffin is then dissolved with a suitable solvent, typically xylene, toluene, or others. These so-called deparaffinizing solvents are then removed with a washing-dehydrating type reagent prior to analysis of the sample by any of the techniques discussed below. Alternatively, slices may be prepared from frozen specimens, fixed briefly in 10% formalin or other suitable fixative, and then infused with dehydrating reagent prior to analysis of the sample.

In a traditional DNA hybridization experiment using a histological sample, formalin-fixed paraffin embedded tissue specimens are cut into sections of 2-6 $\mu$m and collected on slides. The paraffin is melted *(e.g.,* 30-60 minutes at 60°C) and then removed (deparaffinated) by washing with xylene (or a xylene substitute), *e.g*., 2 x 5 minutes. The samples are rehydrated, washed, and then pre-treated (*e.g*., 10 minutes at 95-100°C). The slides are washed and then treated with pepsin or another suitable permeabilizer, *e.g*., 3-15 minutes at 37°C. The slides are washed (*e.g*., 2 x 3 minutes), dehydrated, and probe is applied. The specimens are covered with a coverslip and the slide is optionally incubated at a temperature sufficient to denature any double-stranded nucleic acid in the specimen (*e.g.* 5 minutes at 82°C), followed by incubation at a temperature sufficient to allow hybridization *(e.g.,* overnight at 45°C). After hybridization, the coverslips are removed and the specimens are subjected to a high-stringency wash (*e.g.,* 10 minutes at 65°C) followed by a series of low-stringency washes (*e.g.*, 2 x 3 minutes at room temperature). The samples are then dehydrated and mounted for analysis.

In a traditional RNA hybridization experiment using a histological sample, slides with FFPE tissue sections are deparaffinized in xylene for 2 x 5 min, immerged in 99% ethanol 2 x 3 min, in 96% ethanol 2 x 3 min, and then in pure water for 3 min. Slides are placed in a humidity chamber, Proteinase K is added, and slides are incubated at RT for 5 min- 15 min. Slides are immersed in pure water for 2 x 3 min, immersed in 96% ethanol for 10 sec, and air-dried for 5 min. Probes are added to the tissue section and covered with coverslip. The slides are incubated at 55° C in humidity chamber for 90 min. After incubation, the slides are immersed in a Stringent Wash solution at 55 °C for 25 min, and then immersed in TBS for 10 sec. The slides are incubated in a humidity chamber with antibody for 30 min. The slides are immersed in TBS for 2 x 3 min, then in pure water for 2 x 1 min, and then placed in a humidity chamber. The slides are then incubated with substrate for 60 min, and immersed in tap water for 5 min.

In a traditional northern blot procedure, the RNA target sample is denatured for 10 minutes at 65°C in RNA loading buffer and immediately placed on ice. The gels are loaded and electrophoresed with 1x MOPS buffer (10X MOPS contains 200mM morpholinopropansulfonic acid, 50mM sodium acetate, 10mM EDTA, pH 7.0) at 25 V overnight. The gel is then pre-equilibrated in 20x SSC for 10 min and the RNA is transferred to a nylon membrane using sterile 20x

SSC as transfer buffer. The nucleic acids are then fixed on the membrane using, for example, UV-cross linking at 120 mJ or baking for 30 min at 120°C. The membrane is then washed in water and air dried. The membrane is placed in a sealable plastic bag and prehybridized without probe for 30 min at 68°C. The probe is denatured for 5 min at 100°C and immediately placed on ice. Hybridization buffer (prewarmed to 68°C) is added and the probe is hybridized at 68°C overnight. The membrane is then removed from the bag and washed twice for 5 min each with shaking in a low stringency wash buffer (*e.g.*, 2x SSC, 0.1% SDS) at room temperature. The membrane is then washed twice for 15 min each in prewarmed high stringency wash buffer (*e.g.*, 0.1x SSC, 0.1% SDS) at 68°C. The membrane may then be stored or immediately developed for detection.

Additional examples of traditional hybridization techniques can be found, for example, in Sambrook et al., Molecular Cloning A Laboratory Manual, 2nd Ed., Cold Spring Harbor Laboratory Press, (1989) at sections 1.90-1.104, 2.108-2.117, 4.40-4.41, 7.37-7.57, 8.46-10.38, 11.7-11.8, 11.12-11.19, 11.38, and 11.45-11.57; and in Ausubel et al., Current Protocols in Molecular Biology, John Wiley & Sons, Inc. (1998) at sections 2.9.1-2.9.6, 2.10.4-2.10.5, 2.10.11-2.10.16, 4.6.5-4.6.9, 4.7.2-4.7.3, 4.9.7-4.9.15, 5.9.18, 6.2-6.5, 6.3, 6.4, 6.3.3-6.4.9, 5.9.12-5.9.13, 7.0.9, 8.1.3, 14.3.1-14.3.4, 14.9, 15.0.3-15.0.4, 15.1.1-15.1.8, and 20.1.24-20.1.25.

**(2) Hybridization Techniques**

The methods of the present invention can be used fully or partly in all types of nucleic acid hybridization techniques known in the art for cytological and histological samples. Such techniques include, for example, *in situ* hybridization (ISH), fluorescent *in situ* hybridization (FISH; including multi-color FISH, Fiber-FISH, etc.), chromogenic *in situ* hybridization (CISH), silver *in situ* hybridization (SISH), comparative genome hybridization (CGH), chromosome paints, and arrays *in situ.* The disclosed compositions will improve the efficiency of traditional hybridization techniques, *e.g.*, by eliminating the need for a denaturation step.Molecular probes that are suitable for use in the hybridizations of the invention are described, *e.g.*, in U.S. Patent Publication No. 2005/0266459. In general, probes may be prepared by chemical synthesis, PCR, or by amplifying a specific DNA sequence by cloning, inserting the DNA into a vector, and amplifying the vector an insert in appropriate host cells. Commonly used vectors include bacterial plasmids, cosmids, bacterial artificial chromosomes (BACs), PI diverted artificial chromosomes (PACs), or yeast artificial chromosomes (YACs). The amplified DNA is then extracted and purified for use as a probe. Methods for preparing and/or synthesizing probes are known in the art, *e.g.,* as disclosed in PCT/US02/30573.

In general, the type of probe determines the type of feature one may detect in a hybridization assay. For example, total nuclear or genomic DNA probes can be used as a species-specific probe. Chromosome paints are collections of DNA sequences derived from a single chromosome type and can identify that specific chromosome type in metaphase and interphase nuclei, count the number of a certain chromosome, show translocations, or identify extra-chromosomal fragments of chromatin. Different chromosomal types also have unique repeated sequences that may be targeted for probe hybridization, to detect and count specific chromosomes. Large insert probes may be used to target unique single-copy sequences. With these large probes, the hybridization efficiency is inversely proportional to the probe size. Smaller probes can also be used to detect aberrations such as deletions, amplifications, inversions, duplications, and aneuploidy. For example, differently-colored locus-specific probes can be used to detect translocations via split-signal *in situ* hybridization.

In general, the ability to discriminate between closely related sequences is inversely proportional to the length of the hybridization probe because the difference in thermal stability decreases between wild type and mutant complexes as probe length increases. Probes of greater than 10 bp in length are generally required to obtain the sequence diversity necessary to correctly identify a unique organism or clinical condition of interest. On the other hand, sequence differences as subtle as a single base (point mutation) in very short oligomers (<10 base pairs) can be sufficient to enable the discrimination of the hybridization to complementary nucleic acid target sequences as compared with non-target sequences.

In one embodiment, at least one set of the *in situ* hybridization probes may comprise one or more PNA probes, as defined above and as described in U.S. Patent No. 7,105,294. Methods for synthesizing PNA probes are described in PCT/US02/30573. Alternatively, or in addition, at least one set of the hybridization probes in any of the techniques discussed above may comprise one or more locked nucleic acid (LNA) probes, as described in WO 99/14226. Due to the additional bridging bond between the 2' and 4' carbons, the LNA backbone is pre-organized for hybridization. LNA/DNA and LNA/RNA interactions are stronger than the corresponding DNA/DNA and DNA/RNA interactions, as indicated by a higher melting temperature. Thus, the compositions and methods of the invention, which decrease the energy required for hybridization, are particularly useful for hybridizations with LNA probes.

In one embodiment, the probes may comprise a detectable label (a molecule that provides an analytically identifiable signal that allows the detection of the probe-target hybrid), as described in U.S. Patent Publication No. 2005/0266459. The probes may be labeled to make identification of the probe-target hybrid possible by use, for example, of a fluorescence or bright field microscope/scanner. In some embodiments, the probe may be labeled using radioactive

labels such as $^{31}P$, $^{33}P$, or $^{32}S$, non-radioactive labels such as digoxigenin and biotin, or fluorescent labels. The detectable label may be directly attached to a probe, or indirectly attached to a probe, *e.g.,* by using a linker. Any labeling method known to those in the art, including enzymatic and chemical processes, can be used for labeling probes used in the methods and compositions of the invention. In other embodiments, the probes are not labeled.

In general, *in situ* hybridization techniques such as CGH, FISH, CISH, and SISH, employ large, mainly unspecified, nucleic acid probes that hybridize with varying stringency to genes or gene fragments in the chromosomes of cells. Using large probes renders the *in situ* hybridization technique very sensitive. However, the successful use of large genomic probes in traditional hybridization assays depends on blocking the undesired background staining derived from, *e.g.,* repetitive sequences that are present throughout the genome. Traditional methods for decreasing nonspecific probe binding include saturating the binding sites on proteins and tissue by incubating tissue with prehybridization solutions containing ficoll, bovine serum albumin (BSA), polyvinyl pyrrolidone, and nucleic acids. Such blocking steps are time-consuming and expensive. Advantageously, the methods and compositions of the invention reduce and/or eliminate the need for such blocking steps, and show significantly reduced background levels without the need for blocking agents and without the need for overnight hybridization in formamide-containing buffers. However, in one embodiment, repetitive sequences may be suppressed according to the methods known in the art, *e.g.,* as disclosed in PCT/US02/30573.

Bound probes may be detected in cytological and histological samples either directly or indirectly with fluorochromes *(e.g.,* FISH), organic chromogens *(e.g.,* CISH), silver particles *(e.g.,* SISH), or other metallic particles *(e.g.,* gold-facilitated fluorescence *in situ* hybridization, GOLDFISH). Thus, depending on the method of detection, populations of cells obtained from a sample to be tested may be visualized via fluorescence microscopy or conventional brightfield light microscopy.

Hybridization assays on cytological and histological samples are important tools for determining the number, size, and/or location of specific DNA sequences. For example, in CGH, whole genomes are stained and compared to normal reference genomes for the detection of regions with aberrant copy number. Typically, DNA from subject tissue and from normal control tissue is labeled with different colored probes. The pools of DNA are mixed and added to a metaphase spread of normal chromosomes (or to a microarray chip, for array- or matrix-CGH). The ratios of colors are then compared to identify regions with aberrant copy number.

FISH is typically used when multiple color imaging is required and/or when the protocol calls for quantification of signals. The technique generally entails preparing a cytological sample, labeling probes, denaturing target chromosomes and the probe, hybridizing the probe to the target sequence, and detecting the signal. Typically, the hybridization reaction fluorescently stains the targeted sequences so that their location, size, or number can be determined using fluorescence microscopy, flow cytometry, or other suitable instrumentation. DNA sequences ranging from whole genomes down to several kilobases can be studied using FISH. With enhanced fluorescence microscope techniques, such as, for example, deconvolution, even a single mRNA molecule can be detected. FISH may also be used on metaphase spreads and interphase nuclei.

FISH has been used successfully for mapping repetitive and single-copy DNA sequences on metaphase chromosomes, interphase nuclei, chromatin fibers, and naked DNA molecules, and for chromosome identification and karyotype analysis through the localization of large repeated families, typically the ribosomal DNAs and major tandem array families. One of the most important applications for FISH has been in detecting single-copy DNA sequences, in particular disease related genes in humans and other eukaryotic model species, and the detection of infections agents. FISH may be used to detect, e.g., chromosomal aneuploidy in prenatal diagnoses, hematological cancers, and solid tumors; gene abnormalities such as oncogene amplifications, gene deletions, or gene fusions; chromosomal structural abnormalities such as translocations, duplications, insertions, or inversions; contiguous gene syndromes such as microdeletion syndrome; the genetic effects of various therapies; viral nucleic acids in somatic cells and viral integration sites in chromosomes; etc. In multi-color FISH, each chromosome is stained with a separate color, enabling one to determine the normal chromosomes from which abnormal chromosomes are derived. Such techniques include multiplex FISH (m-FISH), spectral karyotyping (SKY), combined binary ration labeling (COBRA), color-changing karyotyping, cross-species color banding, high resolution multicolor banding, telomeric multiplex FISH (TM-FISH), split-signal FISH (ssFISH), and fusion-signal FISH.

CISH and SISH may be used for many of the same applications as FISH, and have the additional advantage of allowing for analysis of the underlying tissue morphology, for example in histopathology applications. If FISH is performed, the hybridization mixture may contain sets of distinct and balanced pairs of probes, as described in U.S. Patent No. 6,730,474. For CISH, the hybridization mixture may contain at least one set of probes configured for detection with one or more conventional organic chromogens, and for SISH, the hybridization mixture may contain at least one set of probes configured for detection with silver particles, as described in Powell RD et al., "Metallographic in situ hybridization," Hum. Pathol., 38:1145-59 (2007).

The compositions disclosed in the application may also be used fully or partly in all types of molecular biology techniques involving hybridization, including blotting and probing (e.g., Southern, northern, etc.), arrays, and amplification techniques including traditional PCR, RT-PCR, mutational PCR, asymmetric PCR, hot-start PCR, inverse PCR, multiplex

PCR, nested PCR, quantitative PCR, and *in situ* PCR. *In situ* PCR is a polymerase chain reaction that takes place inside a cell on a slide, *e.g.*, the cytology and histology samples described above. Typically, after adhering the sample to a microscope slide, the cells are re-hydrated and permeabilized, and then combined with an appropriate mixture of PCR reagents including polymerase, dNTPs, and primers. The PCR may be carried out in a dedicated instrument, such as the GeneAmp *In situ* PCR System 1000 (Perkin Elmer Biosystems, Foster City, CA) using standard denaturation/renaturation/amplification temperature and time cycles, and the amplified product may be detected using labeled probes or by incorporating labeled dNTPs during the amplification. In some embodiments, the methods of the invention are useful for nucleic acid hybridization applications, with the proviso that such applications do not include amplification of the nucleic acid such as, *e.g.,* by PCR, *in situ* PCR, etc.

**(3) Hybridization Conditions**

The method of the present invention involves the use of polar aprotic solvents in hybridization of nucleic acid chains. The compositions disclosed in the present application are particularly useful for eliminating the denaturation step in said inventive methods.

Hybridization methods using the disclosed compositions may involve applying the compositions to a sample comprising a target nucleic acid sequence, most likely in a double stranded form. The polar aprotic solvent interacts with the double stranded nucleic acids and facilitates their denaturation. Thus, the polar aprotic solvents eliminate the need for a separate denaturation step in hybridization methods.. As a result, the polar aprotic solvents specified in the present invention reduce evaporation of solvents, preserve sample morphology, reduce background, simplify hybridization procedures, and make the hybridization process considerably easier to automate.

Hybridizations using the disclosed compositions may be performed using the same assay methodology as for hybridizations performed with traditional compositions. For example, the heat pre-treatment, digestion, hybridization, washing, and mounting steps may use the same conditions in terms of volumes, temperatures, reagents and incubation times as for traditional compositions. However, the disclosed compositions allow for the elimination of the denaturation step. Additionally, the disclosed compositions allow for reduction of the hybridization time in methods comprising longer hybridization probes or fragments of hybridization probes, for example, hybridization probes or fragments of hybridization probes comprising 40 to 500 nucleotides, hybridization probes or fragments of hybridization probes comprising 50 to 500 nucleotides, or hybridization probes or fragments of hybridization probes comprising 50 to 200 nucleotides. A great variation exists in the traditional hybridization protocols known in the art. For example, some protocols specify a separate denaturation step of potential double stranded nucleotides without probe present, before the following hybridization step, whereas other protocols will denature the probe and sample together. The disclosed compositions may be used in any of traditional hybridization protocols known in the art.

Alternatively, assays using the disclosed compositions can be changed and optimized from traditional methodologies, for example, by decreasing the hybridization time, decreasing the hybridization temperatures, and/or decreasing the hybridization volumes.

For example, in some embodiments, the disclosed compositions will produce strong signals when the denaturation temperature is from 60°C to 100°C and the hybridization temperature is from 20°C to 60°C. In other embodiments, the disclosed compositions will produce strong signals when the denaturation temperature is from 60°C to 65°C, 65°C to 70°C, 70°C to 75°C, 75°C to 80°C, or 80 °C to 85°C, and the hybridization temperature is from 20°C to 30°C, 30°C to 40°C, 40°C to 50°C, or 50°C to 60°C. In other embodiments, the disclosed compositions will produce strong signals when the denaturation temperature is 62 °C, 67°C, 72 °C, or 82°C, and the hybridization temperature is 37°C, 40 °C, 45 °C, 50 °C, or 55°C.

In certain disclosures, the compositions disclosed in the application will produce strong signals when the denaturation time is from 0 to 15 minutes and the hybridization time is from 0 minutes to 24 hours. In other disclosures, the compositions will produce strong signals when the denaturation time is from 0 to 10 minutes and the hybridization time is from 0 minute to 8 hours. In other disclosures, the compositions will produce strong signals when the denaturation time is 0, 1, 2, 3, 4, or 5 minutes, and the hybridization time is 0 minutes, 5 minutes, 15 minutes, 30 minutes, 60 minutes, 180 minutes, or 240 minutes. It will be understood by those skilled in the art that in some cases, e.g., RNA detection, a denaturation step is not required with traditional buffers. It has surprisingly been found that the disclosed compositions also eliminate the need for a denaturation step and/or reduce the temperature required for denaturation of other types of nucleic acids such as, for example, DNA. Thus, in one embodiment, the denaturation time is 0 minutes, *i.e.,* the denaturation step required with prior art buffers is completely eliminated.

Accordingly, hybridizations using the disclosed compositions may be performed in less than 8 hours. In other embodiments, the hybridization step is performed in less than 6 hours. In still other embodiments, the hybridization step is performed within 4 hours. In other embodiments, the hybridization step is performed within 3 hours. In yet other embodiments, the hybridization step is performed within 2 hours. In other embodiments, the hybridization step is performed within 1 hour. In still other embodiments, the hybridization step is performed within 30 minutes. In other embodiments, the hybridization step can take place within 15 minutes. The hybridization step can even take place within 10 minutes or in less than 5 minutes. Figures 1 and 2 illustrate a typical time-course for hybridization applications performed on histological and cytological samples, respectively, using the compositions of the invention compared to hybridization applications using a traditional compositions.

Furthermore, the disclosed compositions allow for fast hybridizations using longer probes or fragments of probes, for example, probes or fragments of probes comprising 40-500 nucleotides, probes or fragments of probes comprising 50-500 nucleotides, or probes or fragments of probes comprising 50-200 nucleotides. In some embodiments, hybridizations may be performed in less than 8 hours. In other embodiments, the hybridization step is performed in less than 6 hours. In still other embodiments, the hybridization step is performed within 4 hours. In other embodiments, the hybridization step is performed within 3 hours. In yet other embodiments, the hybridization step is performed within 2 hours. In other embodiments, the hybridization step is performed within 1 hour. In still other embodiments, the hybridization step is performed within 30 minutes. In other embodiments, the hybridization step can take place within 15 minutes. The hybridization step can even take place within 10 minutes or in less than 5 minutes.

As hybridization time changes, the concentration of probe may also be varied in order to produce strong signals and/or reduce background. For example, as hybridization time decreases, the amount of probe may be increased in order to improve signal intensity. On the other hand, as hybridization time decreases, the amount of probe may be decreased in order to improve background staining.

The disclosed compositions also eliminate the need for a blocking step during hybridization applications by improving signal and background intensity by blocking the binding of, e.g., repetitive sequences to the target DNA. Thus, there is no need to use total human DNA, blocking-PNA, COT-1 DNA, or DNA from any other source as a blocking agent. In addition, the methods of the invention and disclosed compositions surprisingly show significantly reduced background levels without the need for overnight hybridization in formamide-containing buffers. However, background levels can be further reduced by adding agents that reduce non-specific binding, such as to the cell membrane, such as small amounts of total human DNA or non-human-origin DNA *(e.g.,* salmon sperm DNA) to a hybridization reaction using the compositions of the invention.

The aqueous compositions disclosed in the application furthermore provide for the possibility to considerably reduce the concentration of nucleic acid sequences included in the composition. Generally, the concentration of probes may be reduced from 2 to 8-fold compared to traditional concentrations. For example, if HER2 DNA probes and CEN 17 PNA probes are used in the compositions of the invention, their concentrations may be reduced by ¼ and ½, respectively, compared to their concentrations in traditional hybridization compositions. This feature, along with the absence of any requirement for blocking DNA, such as blocking-PNA or COT1, allows for an increased probe volume in automated instrument systems compared to the traditional 10 μL volume used in traditional compositions systems, which reduces loss due to evaporation, as discussed in more detail below.

Reducing probe concentration also reduces background. However, reducing the probe concentration is inversely related to the hybridization time, *i.e*., the lower the concentration, the higher hybridization time required. Nevertheless, even when extremely low concentrations of probe are used with the aqueous compositions of the invention, the hybridization time is still shorter than with traditional compositions.

The disclosed compositions often allow for better signal-to-noise ratios than traditional hybridization compositions. For example, with certain probes, a one hour hybridization with the compositions of the invention will produce similar background and stronger signals than an overnight hybridization in traditional compositions. Background is not seen when no probe is added.

Traditional assay methods may also be changed and optimized when using the disclosed compositions depending on whether the system is manual, semi-automated, or automated. For example, a semi-automated or a fully automated system will benefit from the elimination of a denaturation step that is possible with the compositions of the invention. These changes to traditional hybridization methods may reduce the difficulties encountered when traditional compositions are used in such systems. For example, one problem with semi-automated and fully automated systems is that significant evaporation of the sample can occur during hybridization, since such systems require small sample volumes (*e.g*., 10-150 μL), elevated denaturation temperatures, and extended hybridization times (*e.g.,* 14 hours). Thus, proportions of the components in traditional hybridization compositions are fairly invariable. However, since the disclosed compositions allow for the elimination of a denaturation step, evaporation is reduced, allowing for increased flexibility in the proportions of the components in hybridization compositions used in semi-automated and fully automated systems.

For example, two automated instruments have been used to perform hybridizations using the disclosed compositions in hybridization applications having a traditional denaturation step. Compositions comprising 40% ethylene carbonate (v/v) have been used in the apparatus disclosed in PCT application DK2008/000430, and compositions comprising 15% ethylene carbonate (v/v) have been used in the HYBRIMASTER HS-300 (Aloka CO. LTD, Japan). When the compositions of the invention are used in the HYBRIMASTER HS-300, the instrument can perform rapid FISH hybridization with water in place of the traditional toxic formamide mix, thus improving safety and reducing evaporation. If water wetted strips are attached to the lid of the inner part of the Aloka instrument's reaction unit (hybridization chamber),

*e.g.,* as described in U.S. Patent Application No. 11/031,514, evaporation is reduced even further.

Other problems with automated imaging analysis are the number of images needed, the huge amount of storage place required, and the time required to take the images. The compositions of the invention address this problem by producing very strong signals compared to traditional compositions. Because of the very strong signals produced by the disclosed compositions, the imaging can be done at lower magnification than required for traditional compositions and can still be detected and analyzed, e.g., by algorithms. Since the focal plane becomes wider with lower magnification, the compositions of the invention reduce or eliminate the requirement to take serial sections of a sample. As a result, the overall imaging is much faster, since the disclosed compositions require fewer or no serial sections and each image covers much greater area. In addition, the overall time for analysis is faster, since the total image files are much smaller.

Thus, the methods of the invention solve many of the problems associated with traditional hybridization compositions and methods.

The disclosure may be understood more clearly with the aid of the non-limiting examples that follow, which constitute preferred embodiments of the compositions according to the disclosure. Other than in the examples, or where otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained herein. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope are approximations, the numerical values set forth in the specific example are reported as precisely as possible. Any numerical value, however, inherently contains certain errors necessarily resulting from the standard deviation found in its respective testing measurements. The examples that follow illustrate the present invention.

## EXAMPLES

Reference will now be made in detail to specific embodiments of the invention. While the invention will be described in conjunction with these embodiments, it will be understood that they are not intended to limit the invention to those embodiments. On the contrary, the invention is intended to cover alternatives, modifications, and equivalents, which may be included within the invention as defined by the appended claims.

The reagents used in the following examples are from Dako's Histology FISH Accessory Kit (K5599) and Cytology FISH Accessory Kit (K5499) (Dako Denmark A/S, Glostrup Denmark). The kits contain all the key reagents, except for probe, required to complete a FISH procedure for formalin-fixed, paraffin-embedded tissue section specimens. All samples were prepared according to the manufacturer's description. The Dako Hybridizer (S2451, Dako) was used for the digestion, denaturation, and hybridization steps.

Evaluation of FISH slides was performed within a week after hybridization using a Leica DM6000B fluorescence microscope, equipped with DAPI, FITC, Texas Red single filters and FITC/Texas Red double filter under 10x, 20x, 40x, and 100x oil objective.

Evaluation of CISH slides was performed using an Olympus BX51 light microscope, under 4x, 10x, 20x, 40x, and 60x objective.

In the Examples that follow, "dextran sulfate" refers to the sodium salt of dextran sulfate (D8906, Sigma) having a molecular weight $M_w$ > 500,000. All concentrations of polar aprotic solvents are provided as v/v percentages. Phosphate buffer refers to a phosphate buffered solution containing $NaH_2PO_4$, $2H_2O$ (sodium phosphate dibasic dihydrate) and $Na_2HPO_4$, $H_2O$ (sodium phosphate monobasic monohydrate). Citrate buffer refers to a citrate buffered solution containing sodium citrate ($Na_3C_6H_5O_7$, $2H_2O$; 1.06448, Merck) and citric acid monohydrate ($C_6H_8O_7$, $H_2O$; 1.00244, Merck).

General histology FISH/CISH procedure for Examples 1-20

The slides with cut formalin-fixed paraffin embedded (FFPE) multiple tissue array sections from humans (tonsils, mammacarcinoma, kidney and colon) were baked at 60°C for 30-60 min, deparaffinated in xylene baths, rehydrated in ethanol baths and then transferred to Wash Buffer. The samples were then pre-treated in Pre-Treatment Solution at a minimum of 95°C for 10 min and washed 2 x 3 min. The samples were then digested with Pepsin RTU at 37°C for 3 min, washed 2 x 3 min, dehydrated in a series of ethanol evaporations, and air-dried. The samples were then incubated with 10 μL FISH probe as described under the individual experiments. The samples were then washed by Stringency Wash at 65°C 10 min, then washed 2 x 3 min, then dehydrated in a series of ethanol evaporations, and air-dried. Finally, the slides were mounted with 15 μL Antifade Mounting Medium. When the staining was completed, observers trained to assess signal intensity, morphology, and background of the stained slides performed the scoring.

General cytology FISH procedure for Examples 21-22

Slides with metaphases preparation were fixed in 3.7% formaldehyde for 2 min, washed 2 x 5 min, dehydrated in a series of ethanol evaporations, and air-dried. The samples were then incubated with 10 μL FISH probe as described under the individual experiments. The samples were then washed by Stringency Wash at 65°C 10 min, then washed 2 x 3 min, then dehydrated in a series of ethanol evaporations, and air-dried. Finally, the slides were mounted with 15 μL Antifade Mounting Medium. When the staining was completed, observers trained to assess signal intensity and background of the stained slides performed the scoring as described in the scoring for guidelines for tissue sections.

General histology FISH/CISH procedure for Examples 23-29 and 31-32

Slides with cut formalin-fixed paraffin embedded (FFPE) multiple tissue array sections from humans (tonsils, mammacarcinoma, kidney and colon) were baked at 60°C for 30-60 min, deparaffinated in xylene baths, rehydrated in ethanol baths, and then transferred to Wash Buffer. The samples were then pre-treated in Pre-Treatment Solution at a minimum of 95°C for 10 min and washed 2 x 3 min. The samples were then digested with Pepsin RTU at 37°C for 3 min, washed 2 x 3 min, dehydrated in a series of ethanol evaporations, and air-dried. The samples were then incubated with 10 μL FISH probe as described under the individual experiments. The samples were then washed with Stringency Wash buffer at 65°C 10 min, then washed in Wash Buffer for 2 x 3 min, then dehydrated in a series of ethanol evaporations, and air-dried. Finally, the slides were mounted with 15 μL Antifade Mounting Medium. When the staining was completed, observers trained to assess signal intensity, morphology, and background of the stained slides performed the scoring.

General cytology FISH procedure for Example 30

Slides with metaphase preparations were fixed in 3.7% formaldehyde for 2 min and washed 2 x 5 min. For Example 32, some of the samples were digested with pepsin (Vial 2, K5599, Dako) at 37° C for 2 min and washed 2 x 5 min. All samples were dehydrated in a series of ethanol evaporations and air-dried. The samples were then incubated with 10 μL FISH probe as described under the individual experiments. The samples were then washed in Stringency Wash buffer at 65°C 10 min, then washed in Wash Buffer 2 x 3 min, then dehydrated in a series of ethanol evaporations, and air-dried. Finally, the slides were mounted with 15 μL Antifade Mounting Medium. When the staining was completed, observers trained to assess signal intensity and background of the stained slides performed the scoring as described in the scoring for guidelines for tissue sections.

Scoring Guidelines of tissue sections

The signal intensities were evaluated on a 0-3 scale with 0 meaning no signal and 3 equating to a strong signal. The cell/tissue structures are evaluated on a 0-3 scale with 0 meaning no structure and no nuclei boundaries and 3 equating to intact structure and clear nuclei boundaries. Between 0 and 3 there are additional grades 0.5 apart from which the observer can assess signal intensity, tissue structure, and background.

The signal intensity is scored after a graded system on a 0-3 scale.

0 No signal is seen.
1 The signal intensity is weak.
2 The signal intensity is moderate.
3 The signal intensity is strong.

The scoring system allows the use of ½ grades.

The tissue and nuclear structure is scored after a graded system on a 0-3 scale.

0 The tissue structures and nuclear borders are completely destroyed.
1 The tissue structures and/or nuclear borders are poor. This grade includes situations where some areas have empty nuclei.
2 Tissue structures and/or nuclear borders are seen, but the nuclear borders are unclear. This grade includes situations where a few nuclei are empty.
3 Tissue structures and nuclear borders are intact and clear.

The scoring system allows the use of ½ grades.

The background is scored after a graded system on a 0-3 scale.

0 Little to no background is seen.
1 Some background.
2 Moderate background.
3 High Background.

The scoring system allows the use of ½ grades.

**Example 1**

This example compares the signal intensity and cell morphology from samples treated with the disclosed compositions or traditional hybridization solutions as a function of denaturation temperature.

FISH Probe composition I: 10% dextran sulfate, 300 mM NaCl, 5 mM phosphate buffer, 40% formamide (15515-026, Invitrogen), 5 μM blocking PNAs (*see* Kirsten Vang Nielsen et al., PNA Suppression Method Combined with Fluorescence In Situ Hybridisation (FISH) Technique inPRINS and PNA Technologies in Chromosomal Investigation, Chapter 10 (Franck Pellestor ed.) (Nova Science Publishers, Inc. 2006)), 10 ng/μL Texas Red labeled CCND1 gene DNA probe (RP11-1143E20, size 192 kb).

FISH Probe composition II: 10% dextran sulfate, 300 mM NaCl, 5 mM phosphate buffer, 40% Ethylene carbonate (03519, Fluka), 5 μM blocking PNAs, 10 ng/μL Texas Red labeled CCND1 gene DNA probe (RP11-1143E20, size 192 kb).

Phases of different viscosity, if present, were mixed before use. The FISH probes were denatured as indicated for 5 min and hybridized at 45°C for 60 minutes.

Results:

| Denaturation temperature | Signal | | Cell morphology | |
|---|---|---|---|---|
| | (I) Formamide | (II) EC | Formamide | EC |
| 72°C | 0 | 2 | Good | Good |
| 82°C | ½ | 3 | Good | Good |
| 92°C | ½ | 3 | Not good | Not good |
| *Signals scored as "3" were clearly visible in a 20x objective.* | | | | |

**Example 2**

This example compares the signal intensity and background staining from samples treated with the disclosed compositions or traditional hybridization solutions as a function of hybridization time.

FISH Probe composition I: 10% dextran sulfate, 300 mM NaCl, 5 mM phosphate buffer, 40% formamide, 5 μM blocking PNAs, 10 ng/μL Texas Red labeled CCND1 gene DNA probe.

FISH Probe composition II: 10% dextran sulfate, 300 mM NaCl, 5 mM phosphate buffer, 40% Ethylene carbonate, 5 μM blocking PNAs, 10 ng/μL Texas Red labeled CCND1 gene DNA probe.

Phases of different viscosity, if present, were mixed before use. The FISH probes were incubated at 82°C for 5 min and then at 45°C for 14 hours, 4 hours, 2 hours, 60 minutes, 30 minutes, 15 minutes, 0 minutes.

Results:

| Hybridization time | Signal | | Background staining | |
|---|---|---|---|---|
| | (I) Formamide | (II) EC | Formamide | EC |
| 14 hours | 3 | 3 | +½ | +2 |
| 4 hours | 1 | 3 | +½ | +1 |
| 2 hours | ½ | 3 | +0 | +1 |
| 60 min. | ½ | 3 | +0 | +1 |
| 30 min. | 0 | 2½ | +0 | +1 |
| 15 min. | 0 | 2 | +0 | +1 |

(continued)

| Hybridization time | Signal | | Background staining | |
|---|---|---|---|---|
| | (I) Formamide | (II) EC | Formamide | EC |
| 0 min. | 0 | 1 | +0 | +½ |
| *Signals scored as "3" were clearly visible in a 20x objective.* | | | | |

**Example 3**

This example compares the signal intensity from samples treated with the disclosed compositions having different polar aprotic solvents or traditional hybridization solutions.

FISH Probe composition I: 10% dextran sulfate, 300 mM NaCl, 5 mM phosphate buffer, 40% formamide, 5 μM blocking PNAs, 10 ng/μL Texas Red labeled CCND1 gene DNA probe.

FISH Probe composition II: 10% dextran sulfate, 300 mM NaCl, 5 mM phosphate buffer, 40% Ethylene carbonate (EC), 5 μM blocking PNAs, 10 ng/μL Texas Red labeled CCND1 gene DNA probe.

FISH Probe composition III: 10% dextran sulfate, 300 mM NaCl, 5 mM phosphate buffer, 40% Propylene carbonate (PC) (540013, Aldrich), 5 μM blocking PNAs, 10 ng/μL Texas Red labeled CCND1 gene DNA probe.

FISH Probe composition IV: 10% dextran sulfate, 300 mM NaCl, 5 mM phosphate buffer, 40% Sulfolane (SL) (T22209, Aldrich), 5 μM blocking PNAs, 10 ng/μL Texas Red labeled CCND1 gene DNA probe.

FISH Probe composition V: 10% dextran sulfate, 300 mM NaCl, 5 mM phosphate buffer, 40% Aceto nitrile (AN) (C02CIIX, Lab-Scan), 5 μM blocking PNAs, 10 ng/μL Texas Red labeled CCND1 gene DNA probe.

FISH Probe composition VI: 10% dextran sulfate, 300 mM NaCl, 5 mM phosphate buffer, 40% γ-butyrolactone (GBL) (B103608. Aldrich), 5 μM blocking PNAs, 7,5 ng/μL Texas Red labeled CCND1 gene DNA probe.

Phases of different viscosity, if present, were mixed before use. The FISH probes were incubated at 82°C for 5 min and then at 45°C for 60 minutes.

Results:

| Signal | | | | | |
|---|---|---|---|---|---|
| (I) Formamide | (II) EC | (III) PC | (IV) SL | (V) AN | (VI) GBL |
| ½ | 3 | 3 | 3 | 2 | 3 |
| *Signals scored as "3" were clearly visible in a 20x objective.* | | | | | |

**Example 4**

This example compares the signal intensity from samples treated with the disclosed compositions having different concentrations of polar aprotic solvent.

FISH Probe Compositions: 10% dextran sulfate, 300 mM NaCl, 5 mM phosphate buffer, 10-60% Ethylene carbonate (as indicated), 5 μM blocking PNAs, 7.5 ng/μL Texas Red labeled *IGK*-constant DNA gene probe ((CTD-3050E15, RP11-1083E8; size 227 kb) and 7.5 ng/μL FITC labeled *IGK*-variable gene DNA probe (CTD-2575M21, RP11-122B6, RP11-316G9; size 350 and 429 kb).

Phases of different viscosity, if present, were mixed before use. The FISH probes were incubated at 82°C for 5 min and then at 45°C for 60 minutes.

Results:

| | | Ethylene carbonate (EC) | | | | |
|---|---|---|---|---|---|---|
| | | 10% | 20% | 30% | 40% | 60% |
| Signal intensity | Texas Red | 1½ | 2 | 3 | 3 | 2 |
| | FITC | 1 | 1½ | 2 | 2½ | 2 |
| *Signals scored as "3" were clearly visible in a 20x objective.* | | | | | | |

**Example 5**

This example compares the signal intensity and background intensity from samples treated with the compositions with and without PNA blocking.

FISH Probe Compositions: 10% dextran sulfate, 300 mM NaCl, 5 mM phosphate buffer, 40% Ethylene carbonate, 7.5 ng/μL Texas Red labeled CCND1 gene DNA probe.

Phases of different viscosity, if present, were mixed before use. The FISH probes were incubated at 82°C for 5 min and then at 45°C for 60 minutes.

Results:

| | Ethylene carbonate (EC) | |
|---|---|---|
| | PNA- blocking | Non- PNA blocking |
| Signal intensity | 3 | 3 |
| Background intensity | ½ + | ½ + |
| *Signals scored as "3" were clearly visible in a 20x objective.* | | |

**Example 6**

This example compares the signal intensity from samples treated with the disclosed compositions as a function of probe concentration and hybridization time.

FISH Probe Compositions: 10% dextran sulfate, 300 mM NaCl, 5 mM phosphate buffer, 40% Ethylene carbonate, and 10, 7.5, 5 or 2.5 ng/μL Texas Red labeled CCND1 gene DNA probe (as indicated).

Phases of different viscosity, if present, were mixed before use. The FISH probes were incubated at 82°C for 5 min and then at 45°C for 3 hours, 2 hours and 1 hours.

Results:

| Hybridization time | Signal Intensity | | | |
|---|---|---|---|---|
| | (I) 10 ng/μL | (II) 7.5ng/μL | (III) 5 ng/μL | (IV) 2.5 ng/μL |
| 3 hours | 3 | 3 | 3 | 3 |
| 2 hours | 3 | 3 | 3 | 1 |
| 1 hours | 3 | 3 | 3 | ½ |
| *Signals scored as "3" were clearly visible in a 20x objective.* | | | | |

**Example 7**

This example compares the signal intensity from samples treated with the disclosed compositions as a function of salt, phosphate, and buffer concentrations.

FISH Probe Compositions: 10% dextran sulfate, ([NaCl], [phosphate buffer], [TRIS buffer] as indicated in Results), 40% Ethylene carbonate, 7.5 ng/μL Texas Red labeled CCND1 gene DNA probe.

Phases of different viscosity, if present, were mixed before use. The FISH probes were incubated at 82°C for 5 min and then at 45°C for 60 minutes.

Results:

| | [N aCl] | | |
|---|---|---|---|
| | 300 mM | 100 mM | 0 mM |
| Signal intensity phosphate [0 mM] | 2 | 1 | ½ |
| Signal intensity phosphate [5 mM] | 3 | 2½ | ½ |
| Signal intensity phosphate [35 mM] | - | - | 3 |
| Signal intensity TRIS [40 mM] | - | - | 2 |
| *Signals scored as "3" were clearly visible in a 20x objective.* | | | |

**Example 8**

This example compares the signal intensity from samples treated with the disclosed compositions as a function of dextran sulfate concentration.

FISH Probe Compositions: 0, 1, 2, 5, or 10% dextran sulfate (as indicated), 300 mM NaCl, 5 mM phosphate buffer, 40% Ethylene carbonate, 5 ng/μL Texas Red labeled SIL-TAL1 gene DNA probe (RP1-278O13; size 67 kb) and 6 ng/μL FITC SIL-TAL1 (ICRFc112-112C1794, RP11-184J23, RP11-8J9, CTD-2007B18, 133B9; size 560 kb).

Phases of different viscosity, if present, were mixed before use. The FISH probes were incubated at 82°C for 5 min and then at 45°C for 60 minutes. No blocking.

Results:

| % Dextran Sulfate | Signal Intensity | |
|---|---|---|
| | Texas Red Probe | FITC Probe |
| 0% | 1 | 1 |
| 1% | 1 | 1 |
| 2% | 1½ | 1½ |
| 5% | 2 | 2½ |
| 10% | 2 | 2½ |
| NOTE: this experiment did not produce results scored as "3" because the SIL-TAL1 Texas Red labeled probe is only 67 kb and was from a non-optimized preparation. | | |

**Example 9**

This example compares the signal intensity from samples treated with the disclosed compositions as a function of dextran sulfate, salt, phosphate, and polar aprotic solvent concentrations.

FISH Probe Composition Ia: 34% dextran sulfate, 0 mM NaCl, 0 mM phosphate buffer, 0% ethylene carbonate, 10 ng/μL Texas Red labeled HER2 gene DNA probe (size 218 kb) and 50 nM of FITC-labeled CEN-7 PNA probe.

FISH Probe Composition Ib: 34% dextran sulfate, 300 mM NaCl, 5 mM phosphate buffer, 0% ethylene carbonate, 10 ng/μL Texas Red labeled HER2 gene DNA probe (size 218 kb) and 50 nM of FITC-labeled CEN-7 PNA probe.

FISH Probe Composition Ic: 34% dextran sulfate, 600 mM NaCl, 10 mM phosphate buffer, 0% ethylene carbonate, 10 ng/μL Texas Red labeled HER2 gene DNA probe (size 218 kb) and 50 nM of FITC-labeled CEN-7 PNA probe.

FISH Probe Composition IIa: 32% dextran sulfate, 0 mM NaCl, 0 mM phosphate buffer, 5% ethylene carbonate, 10 ng/μL Texas Red labeled HER2 gene DNA probe (size 218 kb) and 50 nM of FITC-labeled CEN-7 PNA probe.

FISH Probe Composition IIb: 32% dextran sulfate, 300 mM NaCl, 5 mM phosphate buffer, 5% ethylene carbonate, 10 ng/μL Texas Red labeled HER2 gene DNA probe (size 218 kb) and 50 nM of FITC-labeled CEN-7 PNA probe.

FISH Probe Composition IIc: 32% dextran sulfate, 600 mM NaCl, 10 mM phosphate buffer, 5% ethylene carbonate, 10 ng/μL Texas Red labeled HER2 gene DNA probe (size 218 kb) and 50 nM of FITC-labeled CEN-7 PNA probe.

FISH Probe Composition IIIa: 30% dextran sulfate, 0 mM NaCl, 0 mM phosphate buffer, 10% ethylene carbonate, 10 ng/μL Texas Red labeled HER2 gene DNA probe (size 218 kb) and 50 nM of FITC-labeled CEN-7 PNA probe.

FISH Probe Composition IIIb: 30% dextran sulfate, 300 mM NaCl, 5 mM phosphate buffer, 10% ethylene carbonate, 10 ng/μL Texas Red labeled HER2 gene DNA probe (size 218 kb) and 50 nM of FITC-labeled CEN-7 PNA probe.

FISH Probe Composition IIIc: 30% dextran sulfate, 600 mM NaCl, 10 mM phosphate buffer, 10% ethylene carbonate, 10 ng/μL Texas Red labeled HER2 gene DNA probe (size 218 kb) and 50 nM of FITC-labeled CEN-7 PNA probe.

FISH Probe Composition IVa: 28% dextran sulfate, 0 mM NaCl, 0 mM phosphate buffer, 15% ethylene carbonate, 10 ng/μL Texas Red labeled HER2 gene DNA probe (size 218 kb) and 50 nM of FITC-labeled CEN-7 PNA probe.

FISH Probe Composition IVb: 28% dextran sulfate, 300 mM NaCl, 5 mM phosphate buffer, 15% ethylene carbonate, 10 ng/μL Texas Red labeled HER2 gene DNA probe (size 218 kb) and 50 nM of FITC-labeled CEN-7 PNA probe.

FISH Probe Composition IVc: 28% dextran sulfate, 600 mM NaCl, 10 mM phosphate buffer, 15% ethylene carbonate, 10 ng/μL Texas Red labeled HER2 gene DNA probe (size 218 kb) and 50 nM of FITC-labeled CEN-7 PNA probe.

FISH Probe Reference V: Standard sales vial of HER2 PharmDx probe mix (K5331, Dako) containing blocking PNA. Overnight hybridization for 20 hours.

All compositions were present as a single phase. The FISH probes were incubated at 82°C for 5 min and then at 45°C for 60 minutes with no blocking, except for FISH Probe Reference V, which had PNA blocking and was hybridized for 20 hours.

Results:

| | Signal Strength | |
|---|---|---|
| | DNA Probes | PNA Probes |
| Composition Ia | 0 | ½ |
| Composition Ib | 0 | ½ |
| Composition Ic | ½ | 2½ |
| Composition IIa | ½ | 3 |
| Composition IIb | 1 | 2 |
| Composition IIc | ½ | 3 |
| Composition IIIa | 1 | 2 ½ |
| Composition IIIb | 1 ½ | 2 ½ |
| Composition IIIc | 2 | 3 |
| Composition IVa | 2 ½-3 | 3 |
| Composition IVb | 3 | 3 |
| Composition IVc | 3 | 3 |
| Reference V | 2 | 2 ½ |
| NOTE: Composition IVa gave strong DNA signals with no salt. This is not possible with standard FISH compositions, where DNA binding is salt dependent. | | |

**Example 10**

This example compares the signal intensity from samples treated with the disclosed compositions as a function

of polar aprotic solvent and dextran sulfate concentration under high salt (4x normal) conditions.

FISH Probe Composition I: 0% ethylene carbonate, 29% dextran sulfate, 1200 mM NaCl, 20 mM phosphate buffer, 10 ng/μL Texas Red labeled HER2 gene DNA probe and 50 nM of FITC-labeled CEN-7 PNA probe. Composition was a single phase.

FISH Probe Composition II: 5% ethylene carbonate, 27% dextran sulfate, 1200 mM NaCl, 20 mM phosphate buffer, 10 ng/μL Texas Red labeled HER2 gene DNA probe and 50 nM of FITC-labeled CEN-7 PNA probe. Composition was a single phase.

FISH Probe Composition III: 10% ethylene carbonate, 25% dextran sulfate, 1200 mM NaCl, 20 mM phosphate buffer, 10 ng/μL Texas Red labeled HER2 gene DNA probe and 50 nM of FITC-labeled CEN-7 PNA probe. Composition was a single phase.

FISH Probe Composition IV (not tested): 20% ethylene carbonate, 21% dextran sulfate, 1200 mM NaCl, 20 mM phosphate buffer, 10 ng/μL Texas Red labeled HER2 gene DNA probe and 50 nM of FITC-labeled CEN-7 PNA probe. Composition had two phases.

Results:

| | Signal Strength | |
|---|---|---|
| | DNA Probes | PNA Probes |
| Composition I | ½ | 3 |
| Composition II | 2 | 2 ½ |
| Composition III | 3 | 3 |
| Composition IV | - | - |
| Note: Composition II gave good DNA signals with only 5% EC and strong DNA signals with 10% EC. | | |

**Example 11**

This example compares the signal intensity and background from samples treated with different phases of the disclosed compositions.

FISH Probe Composition: 10% dextran sulfate, 300 mM NaCl, 5 mM phosphate buffer, 40% Ethylene carbonate, 8 ng/μL Texas Red labeled HER2 gene DNA probe and 600 nM FITC-labeled CEN-17 PNA probe. The FISH probes were incubated at 82°C for 5 min and then at 45°C for 60 minutes. No blocking.

Results:

| | Signal Intensity | | |
|---|---|---|---|
| | DNA Probe | PNA Probe | Background |
| Upper Phase | 3 | 1 ½ | +2 |
| Lower Phase | 3 | 2 ½ | +1 |
| Mix of Upper and Lower Phases | 2 ½ | 3 | +½ |
| NOTE: the upper phase had more background than the lower phase in these experiments. | | | |

**Example 12**

This example is similar to the previous example, but uses a different DNA probe and GBL instead of EC.

FISH Probe Composition: 10% dextran sulfate, 300 mM NaCl, 5 mM phosphate buffer, 40% GBL, 10 ng/μL Texas Red labeled CCND1 gene DNA probe and 600 nM FITC-labeled CEN-17 PNA probe.

The FISH probes were incubated at 82°C for 5 min and then at 45°C for 60 minutes. No blocking.

Results:

| | Signal Strength | | Background |
|---|---|---|---|
| | DNA Probe | PNA Probe | |
| Top Phase | 3 | 0-½ | +1 ½ |
| Bottom Phase | 2 | ½ | +3 |
| Mixed Phases | 2 ½ | ½ | +2 ½ |

**Example 13**

This example examines the number of phases in the disclosed compositions as a function of polar aprotic solvent and dextran sulfate concentration.

FISH Probe Compositions: 10 or 20% dextran sulfate; 300 mM NaCl; 5 mM phosphate buffer; 0, 5, 10, 15, 20, 25, 30% EC; 10 ng/μL probe.

Results:

| % EC | Number of Phases 10% Dextran | Number of Phases 20% Dextran |
|---|---|---|
| 0 | 1 | 1 |
| 5 | 1 | 1 |
| 10 | 1 | 1 |
| 15 | 1 | 1 |
| 20 | 2 | 2 |
| 25 | 2 | 2 |
| 30 | 2 | 2 |
| NOTE: 15% EC, 20% dextran sulfate produces the nicest high signal intensities of the above one phase solution. Two phases 20% EC has even higher signal intensities than 15%. (Data not shown). | | |

**Example 14**

This example compares the signal intensity and background from samples treated with different disclosed compositions as a function of probe concentration and hybridization time.

FISH Probe Composition I: 10 ng/μL HER2 TxRed labeled DNA probe (standard concentration) and standard concentration of CEN7 FITC labeled PNA probe (50 nM); 15% EC; 20% dextran sulfate; 600 mM NaCl; 10 mM phosphate buffer.

FISH Probe Composition II: 5 ng/μL HER2 TxRed labeled DNA probe (1/2 of standard concentration) and standard concentration (50 nM) of FITC labeled CEN7 PNA probes; 15% EC; 20% dextran sulfate; 600 mM NaCl; 10 mM phosphate buffer.

FISH Probe Composition III: 2.5 ng/μL HER2 TxRed labeled DNA probe (1/4 of standard concentration) and ½ of the standard concentration (25 nM) of CEN7 PNA probes; 15% EC; 20% dextran sulfate; 600 mM NaCl; 10 mM phosphate buffer.

Compositions I-III existed as a single phase. The FISH probes were incubated at 82°C for 5 min and then at 45°C for 3 hours, 2 hours and 1 hours.

Results:

| Hybridization time | Signal Intensity | | | | | | | | |
|---|---|---|---|---|---|---|---|---|---|
| | I | | | II | | | III | | |
| | DNA | PNA | B.G. | DNA | PNA | B.G. | DNA | PNA | B.G. |
| 3 hours | 3 | 3 | +3 | 3 | 3 | +2.5 | 3 | 3 | +1.5 |
| 2 hours | 2.5 | 2.5 | +3 | 3 | 3 | +3 | 3 | 3 | +1.5 |
| 1 hours | 2.5 | 2.5 | +3 | 3 | 3 | +1.5 | 2.5 | 3 | +1 |
| *Signals scored as "3" were clearly visible in a 20x objective. B.G.: Back ground.* | | | | | | | | | |

**Example 15**

This example compares the signal intensity and background from samples treated with the disclosed compositions as a function of blocking agent.

FISH Probe Compositions: 15% EC; 20% dextran sulfate; 600 mM NaCl; 10 mM phosphate buffer; 2.5 ng/μL HER2 TxRed labeled DNA probe (1/4 of standard concentration) and ½ of the standard concentration (300 nM) FITC labeled CEN17 PNA probe. Samples were blocked with: (a) nothing; (b) 0.1 μg/μL COT1 (15279-011, Invitrogen); (c) 0.3 μg/μL COT1; or (d) 0.1 μg/μL total human DNA before hybridization using the compositions of the invention.

All samples were present as a single phase. The FISH probes were incubated at 82°C for 5 min and then at 45°C for 60 minutes.

Results:

| Blocking Agent | Background | Signal Intensity | |
|---|---|---|---|
| | | DNA | PNA |
| Nothing | +1-1.5 | 3 | 2.5 |
| 0.1 μg/μL COT1 | +1 | 3 | 2.5 |
| 0.3 μg/μL COT1 | +1.5 | 3 | 2.5 |
| 0.1 μg/μL total human DNA | +½ | 3 | 2.5 |
| NOTE: Background levels without blocking are significantly lower than what is normally observed by standard FISH with no blocking. In contrast, if a standard FISH composition does not contain a blocking agent, signals normally cannot be read. | | | |

**Example 16**

This experiment compares different ways of removing background staining using the disclosed compositions.

All compositions contained 15% EC, 20% dextran sulfate, 600 mM NaCl, 10 mM phosphate buffer, 2.5 ng/μL HER2 DNA probes (1/4 of standard concentration), 300 nM CEN17 PNA probe (1/2 of standard concentration), and one of the following background-reducing agents:

A) 5 μM blocking-PNA (*see* Kirsten Vang Nielsen et al., PNA Suppression Method Combined with Fluorescence In Situ Hybridisation (FISH) Technique inPRINS and PNA Technologies in Chromosomal Investigation, Chapter 10 (Franck Pellestor ed.) (Nova Science Publishers, Inc. 2006))
B) 0.1 μg/μL COT-1 DNA
C) 0.1 μg/μL total human DNA (THD) (sonicated unlabelled THD)
D) 0.1 μg/μL sheared salmon sperm DNA (AM9680, Ambion)
E) 0.1 μg/μL calf thymus DNA (D8661, Sigma)
F) 0.1 μg/μL herring sperm DNA (D7290, Sigma)
G) 0.5% formamide
H) 2% formamide
I) 1% ethylene glycol (1.09621, Merck)
J) 1% glycerol (1.04095, Merck)

K) 1% 1,3-Propanediol (533734, Aldrich)
L) 1% $H_20$ (control)

All samples were present as a single phase. The probes were incubated at 82°C for 5 minutes and then at 45°C on FFPE tissue sections for 60 and 120 minutes.

Results:

| Background blocking | Hybridization/min | Background | Signal Intensity DNA | PNA |
|---|---|---|---|---|
| Blocking-PNA | 60 | +1 | 3 | 2.5 |
| Blocking-PNA | 120 | +1-1½ | 3 | 2.5 |
| COT-1 | 60 | +½ | 3 | 2.5 |
| COT-1 | 120 | +0-½ | 3 | 2.5 |
| THD | 60 | +0 | 3 | 3 |
| THD | 120 | +½ | 3 | 2.5 |
| Salmon DNA sperm | 60 | +0 | 3 | 3 |
| Salmon DNA sperm | 120 | +0 | 3 | 3 |
| Calf Thymus DNA | 60 | +0 | 2.5 | 3 |
| Calf Thymus DNA | 120 | +½ | 3 | 2.5 |
| Hearing sperm DNA | 60 | +0 | 3 | 3 |
| Hearing sperm DNA | 120 | +½ | 2.5 | 3 |
| 0.5% formamide | 60 | +0 | 2.5 | 3 |
| 0.5% formamide | 120 | +0 | 3 | 3 |
| 2% formamide | 60 | +½ | 2.5 | 3 |
| 2% formamide | 120 | +½ | 3 | 3 |
| 1 % Ethylene Glycol | 60 | +½ | 2.5 | 3 |
| 1% Ethylene Glycol | 120 | +1½ | 3 | 2.5 |
| 1 % Glycerol | 60 | +½ | 0.5 | 3 |
| 1 % Glycerol | 120 | +1 | 3 | 2.5 |
| 1% 1,3-Propanediol | 60 | +0 | 3 | 2.5 |
| 1% 1,3-Propanediol | 120 | +1 | 3 | 2.5 |
| Nothing | 60 | +1 | 2.5 | 2.5 |
| Nothing | 120 | +1½ | 3 | 2.5 |
| NOTE: all background reducing reagents, except for blocking-PNA, showed an effect in background reduction. Thus, specific blocking against repetitive DNA sequences is not required. | | | | |

**Example 17**

This experiment compares the signal intensity from the upper and lower phases using two different polar aprotic solvents.

FISH Probe Composition I: 10% dextran sulfate, 300 mM NaCl, 5 mM phosphate buffer, 40% ethylene trithiocarbonate (ET) (E27750, Aldrich), 5 μM blocking PNAs, 10 ng/μL Texas Red labeled CCND1 gene DNA probe.

FISH Probe Composition II: 10% dextran sulfate, 300 mM NaCl, 5 mM phosphate buffer, 40% glycol sulfite (GS) (G7208, Aldrich), 5 μM blocking PNAs, 10 ng/μL Texas Red labeled CCND1 gene DNA probe.

The FISH probes were incubated at 82°C for 5 min and then at 45°C for 60 minutes.

Results:

| | Signal Intensity | |
|---|---|---|
| | I (ET) | II (GS) |
| Upper Phase | 1 ½ | 0 |
| Lower Phase | 0 | 315 |
| Mix of Upper and Lower Phases | 2 ½ | 3 |

**Example 18.**

This experiment examines the ability of various polar aprotic solvents to form a one-phase system.

All compositions contained: 20% dextran sulfate, 600 mM NaCl, 10 mM phosphate buffer, and either 10, 15, 20, or 25% of one of the following polar aprotic solvents:

Sulfolane
γ-Butyrolactone
Ethylene trithiocarbonate
Glycol sulfite
Propylene carbonate

Results: all of the polar aprotic solvents at all of the concentrations examined produced at least a two-phase system in the compositions used. However, this does not exclude that these compounds can produce a one-phase system under other composition conditions.

**Example 19**

This experiment examines the use of the disclosed compositions in chromogenic *in situ* hybridization (CISH) analysis on multi FFPE tissue sections.

FISH Probe Composition I: 4.5 ng/μL TCRAD FITC labelled gene DNA probe (1/4 of standard concentration) (RP11-654A2, RP11-246A2, CTP-2355L21, RP11-158G6, RP11-780M2, RP11-481C14; size 1018 kb); 15% EC; 20% dextran sulfate; 600 mM NaCl; 10 mM citrate buffer, pH 6.0.

FISH Probe Composition II: 4.5 ng/μL TCRAD FITC labelled gene DNA probe (1/4 of standard concentration) (size 1018 kb); 15% EC; 20% dextran sulfate; 600 mM NaCl; 10 mM citrate buffer, pH 6.0; 0.1 ug/uL sheared salmon DNA sperm.

FISH Probe Composition III: 300 nM of each individual FITC labelled PNA CEN17 probe (1/2 of standard concentration); 15% EC; 20% dextran sulfate; 600 mM NaCl; 10 mM citrate buffer, pH 6.0.

All samples were analyzed using the Dako DuoCISH protocol (SK108) and compositions for split probes with the exception that the stringency wash was conducted for 20 minutes instead of 10 minutes, and without using the DuoCISH red chromogen step.

Results:

| | Signal Strength | |
|---|---|---|
| Composition | FITC DNA | FITC PNA |
| I | 3 | - |
| II | 3 | - |

(continued)

| | Signal Strength | |
|---|---|---|
| Composition | FITC DNA | FITC PNA |
| III | - | 3 |
| Note: The signal intensities were very strong. Due to the high levels of background, it was not possible to discriminate if addition of salmon sperm DNA in Composition II reduced the background. *Signals were clearly visible using a 10x objective in e.g. tonsils, which in general had less background. If tissues possessed high background, the signals were clearly visible using a 20x objective.* | | |

**Example 20**

This example compares the signal intensity and background from FFPE tissue sections treated with the disclosed compositions with two DNA probes.

FISH Probe Composition I: 9 ng/μL IGH FITC labelled gene DNA probe (RP11-151B17, RP11-112H5, RP11-101G24, RP11-12F16, RP11-47P23, CTP-3087C18; size 612 kb); 6.4 ng/μL MYC Tx Red labeled DNA probe (CTD-2106F24, CTD-2151C21, CTD-2267H22; size 418 kb); 15% EC; 20% dextran sulfate; 600 mM NaCl; 10 mM citrate buffer, pH 6.0.

FISH Probe Composition II: 9 ng/μL IGH FITC labelled gene DNA probe; 6.4 ng MYC TxRed labeled DNA probe; 15% EC, 20% dextran sulfate; 600 mM NaCl; 10 mM citrate buffer, pH 6.0; 0.1 ug/uL sheared salmon sperm DNA.

| | Signal Strength | | |
|---|---|---|---|
| Salmon DNA | FITC probe | Texas Red probe | Background |
| - | 2 ½ | 2 ½ | +2.5 |
| + | 3 | 3 | +1.5 |
| NOTE: the high background was probably due to the fact that standard probe concentrations were used. | | | |

**Example 21**

This experiment examines the use of the disclosed compositions on cytological samples.

FISH Probe Composition: 15% EC; 20% dextran sulfate; 600 mM NaCl; 10 mM phosphate buffer; 5 ng/μL HER2 TxRed labeled DNA probe (1/2 of standard concentration) and ½ of the standard concentration of CEN7 (25 nM).

The FISH probes were incubated on metaphase chromosome spreads at 82°C for 5 minutes, then at 45°C for 30 minutes, all without blocking.

Results:

| Signal Strength | | Background |
|---|---|---|
| DNA Probe | PNA Probe | |
| 3 | 3 | +1 |

No chromosome banding (R-banding pattern) was observed with the compositions of the invention, in contrast with traditional ISH solutions, which typically show R-banding. A low homogenously red background staining of the interphase nuclei and metaphase chromosomes was observed.

**Example 22**

This example compares the signal intensity and background from DNA probes on cytology samples, metaphase spreads, with and without blocking.

FISH Probe Composition I: 6 ng/μL TCRAD Texas Red labelled gene DNA probe (standard concentration) (CTP-31666K20, CTP-2373N7; size 301 kb) and 4.5 ng/μL FITC labelled gene DNA probe (1/4 of standard concentration); 15% EC, 20% dextran sulfate; 600 mM NaCl; 10 mM citrate buffer, pH 6.0.

FISH Probe Composition II: 6 ng/μL TCRAD Texas Red labelled gene DNA probe (standard concentration) (size 301 kb) and 4.5 ng/μL FITC labelled gene DNA probe (1/4 of standard concentration); 15% EC, 20% dextran sulfate; 600 mM NaCl; 10 mM citrate buffer, pH 6.0; 0.1 ug/uL sheared salmon sperm DNA.

The FISH probes were incubated on metaphase spreads at 82°C for 5 min, then at 45°C for 60 min.

Results:

| Blocking Agent | Background | Signal Intensity Tx Red | FITC |
|---|---|---|---|
| Nothing | +0 | 3 | 3 |
| 0.1 μg/μL Salmon DNA | +0 | 3 | 3 |

Again, no chromosome banding (R-banding pattern) was observed with the compositions of the invention. In addition, no background staining of the interphase nuclei or the metaphase chromosomes were observed.

**Comparative Example 23**

This example compares signal intensity and background as a function of denaturation at different temperatures and times.

FISH Probe Composition: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The slides were denaturated as indicated in the table and hybridized at 45° C for 60 min.

Results:

| Denaturation temperature | Denaturation time | Background | Signal Intensity DNA | PNA |
|---|---|---|---|---|
| 82° C | 5 min | +3 | 3 | 3 |
| 82° C | 10 min | +2½ | 3 | 3 |
| 72° C | 10 min | +1½ | 3 | 3 |
| 62° C | 10 min | +½ | 2½-3 | 3 |

These results show that background was significantly lower when samples were denaturated at 72° C and 62° C for 10 min., compared to 82° C for 5 and 10 min. Thus, the compositions of the invention produce strong signals with improved background at lower denaturation temperatures.

**Comparative Example 24**

This example compares signal intensity and background as a function of denaturation at different temperatures and times.

FISH Probe Composition: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The slides were denaturated as indicated in the table and hybridized at 45° C for 60 min.

Results:

| Denaturation temperature | Denaturation time | Background | Signal Intensity DNA | PNA |
|---|---|---|---|---|
| 82° C | 5 min | +2½ | 3 | 3 |
| 72° C | 10 min | +1 | 3 | 3 |
| 67° C | 10 min | +½ | 3 | 3 |
| 62°C | 10 min | +1 | 3 | 3 |

These results show that background was significantly lower when samples were denaturated at 72, 67 and 62° C for 10 min., compared to 82° C for 5 min. Thus, the compositions of the invention produce strong signals with improved background at lower denaturation temperatures.

**Comparative Example 25**

This example compares signal intensity and background as a function of denaturation at different temperatures and times.

FISH Probe Composition: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The slides were denaturated as indicated in the table and hybridized at 45° C for 60 min.

Results:

| Denaturation temperature | Denaturation time | Background | Signal Intensity DNA | PNA |
|---|---|---|---|---|
| 82° C | 5 min | +2 | 3 | 3 |
| 72° C | 15 min | +1 | 3 | 2½ |
| 67° C | 15 min | +3 | 3 | 2½ |
| 62° C | 15 min | +3 | 3 | 3 |

These results show that the background was higher when samples were denaturated at 67 and 62° C for 15 min. However, the type of background observed (green lines across the tissue as opposed to the more normal reddish background), suggested that sample morphology began to suffer from the 15 minute denaturation. This type of background may be reduced by using milder heat pre-treatment and/or pepsin digestion conditions.

In general, lower denaturation temperatures decrease background levels significantly. In addition, lowering the denaturation temperature, but keeping the time of 5 min., decreases the signal intensity of the DNA probe (data not shown).

**Comparative Example 26**

This example compares signal intensity and background as a function of hybridization at different temperatures when samples are denaturated at 82° C for 5 min.

FISH Probe Composition I: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

FISH Probe Composition II: 15% EC, 20% dextran sulfate; 600 mM NaCl; 10 mM citrate buffer, pH 6.0

The slides were denaturated with FISH probe compositions at 82° C for 5 min. and hybridized as indicated in the table for 60 min.

Results:

| Hybridization temperature | FISH probe composition | Background | Signal Intensity DNA | PNA |
|---|---|---|---|---|
| 45° C | I | +1-2 | 2½-3 | 2½ |
| 50° C | I | +1-1½ | 3 | 2½ |
| 55° C | I | +1½-2 | 2-2½ | 2½ |
| 45° C | II | +0 | 0 | 0 |

These results show that the strongest signals were observed at 50°C hybridization. The control without probe showed no background staining. The morphology began to suffer when hybridized at 55°C. However, this effect on morphology may be reduced by using milder heat pre-treatment and/or pepsin digestion conditions.

**Comparative Example 27**

This example compares signal intensity and background as a function of hybridization at different temperatures when samples are denaturated at 67° C for 10 min.

FISH Probe Composition: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The slides were denaturated with the FISH probe composition at 67° C for 10 min. and hybridized for 60 min.

Results:

| Hybridization temperature | Background | Signal Intensity DNA | PNA |
|---|---|---|---|
| 45° C | +1-1½ | 3 | 2½ |
| 50° C | +1-2 | 3 | 3 |
| 55° C | +1-2 | 3 | 2½ |

These results show that the strongest signals were observed at 50° C hybridization. The morphology began to suffer when hybridized at 55°C. However, this effect on morphology may be reduced by using milder heat pre-treatment and/or pepsin digestion conditions.

**Example 28**

This example compares signal intensity and background from hybridization at 45 °C and at room temperature (RT, 21°C) without denaturing the probe and tissue.

FISH Probe Composition: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The FISH probes and tissue (not denaturated) were hybridized at RT in a humidity chamber or in Dako Hybridizer (S2450, Dako) at 45°C overnight.

Results:

| Denaturation | Hybridization temperature | Tissue | Background | Signal Intensity DNA | PNA |
|---|---|---|---|---|---|
| non | RT | Mamma | +0 | 0 | 2½ |
| non | RT | Tonsils | +0 | 0 | 2½ |
| non | 45° C | Mamma | +0-1½ | 3 | 3 |
| non | 45°C | Tonsils | +0 | 3 | 3 |

These results show that the buffer can be used without denaturating either the specimen or the probe composition. Note that the probe was not heated above RT at any point before or during this experiment, aside from the hybridization step.

**Example 29**

This example compares the background and signal intensity from hybridization at 45 and 50°C (for 1 h and O/N) without denaturation of the tissue section, and with or without denaturation of the FISH probe (comparative example to the extent that denaturing took place).

FISH Probe Composition I: 3.3 ng/μL HER2 TxRed labeled DNA probe (1/3 of standard concentration) and ½ of the standard concentration (300 nM) of FITC labeled CEN17 PNA probes; 15% EC, 20% dextran sulfate; 600 mM NaCl; 10 mM citrate buffer, pH 6.0.
FISH Probe Composition II (HER2 PharmDx probe mix, K5331, Dako): 10 ng/μL HER2 TxRed labeled DNA probe and (600 nM) of FITC labeled CEN17 PNA probes, 45% formamide, 10% dextran sulfate, 300 mM NaCl, 5 mM Phosphate buffer, 5 μM unlabelled blocking PNAs.

The slides were pre-treated as described above in the standard Dako FISH protocol until after the dehydration step (K5599, Dako). The FISH probe was either not heat denaturated, or heat denaturated on a heat block in 1.5 mL centrifuge tubes at 67° C for 1 min or at 82° C for 5 min. and put on ice. The samples were pre-treated, dehydrated, and air-dried. Ten μL of non heat denaturated or heat denaturated FISH probe was added to the sample, coverslipped and sealed, and hybridized at 45° C for 60 min. Then the standard Dako FISH procedure was followed. The probe was not heated above RT at any point before the experiment.

Results:

| Probe | Probe denaturation temp./time | Hybridization temp. | Hybridization time | Background | Signal Intensity DNA | PNA |
|---|---|---|---|---|---|---|
| I | - | 45° C | 1h | +0-½ | 2 | 3 |
| II | - | 45°C | 1h | 0 | 0 | 3 |
| I | - | 45° C | O/N* | +1-2 | 3 | 2-2½ |
| II | - | 45°C | O/N | +0 | ½ | 2½ |
| I | - | 50° C | 1h | +½ | 2½ | 3 |
| II | - | 50° C | 1h | +0 | 0 | 3 |
| I | - | 50° C | O/N* | +2-3 | 3 | 1½ |
| II | - | 50° C | O/N | +0 | ½ | 2 |
| I | 67° C/1 min | 45° C | 1h | +½ | 2½-3 | 3 |
| II | 67° C/1 min | 45°C | 1h | 0 | 0 | 3 |
| I | 67° C/1 min | 45° C | O/N* | +2 | 3 | 2 |
| II | 67° C/1 min | 45°C | O/N | +0 | 1½-2 | 2-2½ |
| I | 67° C/1 min | 50° C | 1h | +1 | 2½-3 | |
| II | 67° C/1 min | 50° C | 1h | +0 | 0 | 2½ |
| I | 67° C/1 min | 50° C | O/N* | +2 | 3 | 2 |
| II | 67° C/1 min | 50° C | O/N | +1 | 1 | 2½ |
| I | 82° C/5 min | 45° C | 1h | +½-1 | 2-2½ | 3 |
| II | 82° C/5 min | 45°C | 1h | 0 | 0 | 2 |
| I | 82° C/5 min | 45° C | O/N* | +2-3 | 3 | 1½ |
| II | 82° C/5 min | 45°C | O/N | +0 | 1 | 2 |
| I | 82° C/5 min | 50° C | 1h | +1-1½ | 3 | 2-2½ |

(continued)

| Probe | Probe denaturation temp./time | Hybridization temp. | Hybridization time | Background | Signal Intensity DNA | PNA |
|---|---|---|---|---|---|---|
| n | 82° C/5 min | 50° C | 1h | 0 | 0 | 2 |
| I | 82° C/5 min | 50° C | O/N* | +3 | 2 | 1 |
| II | 82° C/5 min | 50° C | O/N | 0 | 1 | 3 |
| *The slides with FISH Probe Composition I were dried out after O/N hybridization. | | | | | | |

These results show that DNA and PNA based probes do not require denaturation of the tissue specimen or the probe with FISH probe composition I. However, if FISH probe composition I was denaturated at 67° C for 1 min or 82° C for 5 min prior to hybridization, stronger signals were obtained. The compositions of the invention showed improved DNA signals with no denaturation of the sample with or without denaturation of the probe, compared to formamide-containing buffers. Note that for FISH probe composition II (formamide), the scorings for the DNA based probe were all zero at hybridization incubation for 60 min. Thus, the compositions of the invention showed signals at one hour hybridizations without denaturation.

**Example 30**

The example compares the signal intensity on cytological specimens (metaphase spreads) from hybridization at 45 °C without denaturing the probe and tissue.

FISH Probe Composition I: 2.5 ng/μL HER2 TxRed labeled DNA probe (1/4 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

FISH Probe Composition II (HER2 PharmDx probe mix, K5331, Dako): 10 ng/μL HER2 TxRed labeled DNA probe and (600 nM) of FITC labeled CEN17 PNA probes, 45% formamide, 10% dextran sulfate, 300 mM NaCl, 5 mM Phosphate buffer, 5 μM unlabelled blocking PNAs.

After formaldehyde fixation, some of the specimens were digested with pepsin (Vial 2, K5599) to allow better access for the non-heated probe to the non-heated target. Pepsin was applied at 37°C for 2 min., then washed 2 x 5 min. with Wash Buffer, before performing the dehydration step. The FISH probes and metaphase spreads (not denaturated) were hybridized at 45° C for 180 min. The control sample was denaturated at 82° C for 5 min, followed by hybridization at 45° C for 180 min. The probe was not heated above RT at any point before the experiment

Results:

| Probe | Pepsin | Denaturation | Signal Intensity DNA | PNA |
|---|---|---|---|---|
| I | - | - | ½ | 1 |
| I | + | - | 1½ | 2 |
| I | - | + | 2½ | 2½ |
| II | - | - | 0 | 0 |
| II | + | - | 0 | ½ |
| II | - | + | 1 | 3 |

These results show that the compositions of the invention (Composition I) produce stronger signals than traditional formamide-containing compositions (Composition II) when the sample and probe are not denatured, and when the samples are digested with pepsin. In addition, the compositions of the invention produce stronger signals for DNA probes than traditional formamide-containing compositions even when a denaturation step is performed.

Note that the above experiment involved a 3 hour hybridization. In other experiments, overnight hybridization of metaphase specimens created high background staining, and 60 min hybridization showed weak signals (data not

shown). Note also that the structure of the chromosomes in the metaphase spreads that had not been denaturated was better conserved than those that had been denaturated. This was true for both Composition I and II.

**Example 31**

This example compares the signal intensity and background from hybridization of FISH probes on FFPE tissue sections hybridized at 50°C for 120 min or denaturated at 82° C for 5 min and hybridized at 45° C for 60 min (comparative example to the extent that denaturing took place).

FISH Probe Composition I: 3.3 ng/μL HER2 TxRed labeled DNA probe (1/3 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

FISH Probe Composition II: 3 ng/μL TOP2A TxRed labeled DNA probe (1/3 of standard concentration) and ½ of the standard concentration (300 nM) of FITC labeled CEN17 PNA probes; 15% EC, 20% dextran sulfate; 600 mM NaCl; 10 mM citrate buffer, pH 6.0.

The probe was not heated above RT at any point before the experiment.

Results:

| Probe | Denaturation temp./time | Hybridization temp./time | Sample* | Background** | Signal Intensity DNA | Signal Intensity PNA |
|---|---|---|---|---|---|---|
| HER2 (I) | - | 50° C/120 min | Mamacarcinoma | +0 | 2½-3 | 2½-3 |
| HER2 (I) | - | 50° C/120 min | Tonsils | +0 | 2-3 | 3 |
| HER2 (I) | - | 50° C/120 min | Kidney | +½ | 2 | 2½ |
| HER2 (I) | - | 50° C/120 min | Colon | +0 | 1½ -2 | 2-2½ |
| TOP2A (II) | - | 50° C/120 min | Mamacarcinoma | +0 | 2½ | 3 |
| TOP2A (II) | - | 50° C/120 min | Tonsils | +0 | 2-2½ | 3 |
| TOP2A (II) | - | 50° C/120 min | Kidney | +2½ | 1½-2 | 2-2½ |
| TOP2A (II) | - | 50° C/120 min | Colon | +0 | 1½ | 2-2½ |
| HER2 (I) | 82° C/5 min | 45°C/60 min | Mamacarcinoma | +2½ | 2½ | 2½ |
| HER2 (I) | 82° C/5 min | 45°C/60 min | Tonsils | +2½ | 2½ | 2 |
| HER2 (I) | 82° C/5 min | 45°C/60 min | Kidney | +2½ | 1½ -2 | 1½-2 |
| HER2 (I) | 82° C/5 min | 45°C/60 min | Colon | +2½ | 1½-2 | 1½-2 |
| TOP2A (II) | 82° C/5 min | 45°C/60 min | Mamacarcinoma | +1 | 2½ | 3 |
| TOP2A (II) | 82° C/5 min | 45°C/60 min | Tonsils | +1 | 2½-3 | 2 |
| TOP2A (II) | 82° C/5 min | 45°C/60 min | Kidney | +1½ | 1½-2 | 1½-2 |
| TOP2A (II) | 82° C/5 min | 45°C/60 min | Colon | +1 | 1½-2 | 2-2½ |

Colon and kidney provided weaker signals than mamacarcinoma and tonsils. It is observed in (F)ISH that tissues like, *e.g.,* colon and kidney, might require a longer pre-treatment than, *e.g.,* mammacarcinoma and tonsils to obtain strong signals, *e.g.,* by longer pepsin digestion incubation, because of both the specific characteristics of the tissue and most importantly often longer tissue fixation time *(i.e.,* harsher pre-treatment). Note, that the signal intensity of the colon and kidney with no denaturation is equivalent with denaturated tissue and probe. Thus, these results surprisingly illustrate that the compositions of the invention eliminate the need for a heat denaturation step for the tissue samples.

**Example 32**

This example compares the background and signal intensity from hybridizations performed with sulfolane, propylene carbonate, γ-butyrolactone with and without denaturation (comparative example to the extent that denaturing took place).

FISH Probe composition I: 40% SL (T22209, Aldrich), 10% dextran sulfate, 300 mM NaCl, 5 mM phosphate buffer, 5 ng/μL HER2 TxRed labeled DNA probe (1/2 of standard concentration) and the standard concentration (600 nM) of FITC labeled CEN17 PNA probes.

FISH Probe composition II: 15% SL, 20% dextran sulfate; 600 mM NaCl; 10 mM citrate buffer, pH 6.0, 5 ng/μL HER2 TxRed labeled DNA probe (1/2 of standard concentration) and the standard concentration (600 nM) of FITC labeled CEN17 PNA probes.

FISH Probe Composition III: 40% PC (540013, Aldrich), 10% dextran sulfate, 300 mM NaCl, 5 mM phosphate buffer, 5 ng/μL HER2 TxRed labeled DNA probe (1/2 of standard concentration) and the standard concentration (600 nM) of FITC labeled CEN17 PNA probes.

FISH Probe composition IV: 15% PC, 20% dextran sulfate; 600 mM NaCl; 10 mM citrate buffer, pH 6.0, 5 ng/μL HER2 TxRed labeled DNA probe (1/2 of standard concentration) and the standard concentration (600 nM) of FITC labeled CEN17 PNA probes.

FISH Probe composition V: 40% GBL (B103608, Aldrich), 10% dextran sulfate, 300 mM NaCl, 5 mM phosphate buffer, 5 ng/μL HER2 TxRed labeled DNA probe (1/2 of standard concentration) and the standard concentration (600 nM) of FITC labeled CEN17 PNA probes.

FISH Probe composition VI: 15% GBL, 20% dextran sulfate; 600 mM NaCl; 10 mM citrate buffer, pH 6.0, 5 ng/μL HER2 TxRed labeled DNA probe (1/2 of standard concentration) and the standard concentration (600 nM) of FITC labeled CEN17 PNA probes.

FISH Probe composition VII: 15% EC, 20% dextran sulfate; 600 mM NaCl; 10 mM citrate buffer, pH 6.0, 3.3 ng/μL HER2 TxRed labeled DNA probe (1/3 of standard concentration) and ½ of the standard concentration (300 nM) of FITC labeled CEN17 PNA probes.

FISH Probe composition VIII: 15% formamide (FM)(15515-026, Invitrogen), 20% dextran sulfate; 600 mM NaCl; 10 mM citrate buffer, pH 6.0, 5 ng/μL HER2 TxRed labeled DNA probe (1/2 of standard concentration) and the standard concentration (600 nM) of FITC labeled CEN17 PNA probes.

The FISH compositions I-VI had two phases at room temperature. For the 40% compositions (I, III and V), only the top phase of the two phases was used. The 15% compositions that displayed phases of different viscosity (II, IV, VI) were mixed before use. Compositions VII and VIII had one phase.

Results:

| Probe | Denaturation temp./time | Hybridization temp./time | Sample* | Background** | Signal Intensity DNA | Signal Intensity PNA |
|---|---|---|---|---|---|---|
| SL 40% (I)* | - | 50° C/120 min | Mamacarcinoma<br>Tonsils | +2½<br>+2½ | 2-2½<br>1½-2½ | ½<br>½ |
| SL 40% (I)* | 82° C/5 min | 45° C/60 min | Mamacarcinoma<br>Tonsils | +2<br>+2 | 2-2½<br>1-1½ | ½<br>½ |
| SL 15% (II) | - | 50° C/120 min | Mamacarcinoma<br>Tonsils | +2<br>+1 | 2-2½<br>3 | 2-2½<br>2½-3 |
| SL 15% (II) | 82° C/5 min | 45° C/60 min | Mamacarcinoma<br>Tonsils | +1<br>+0 | 2-2½<br>3 | 2-2½<br>2-2½ |

(continued)

| Probe | Denaturation temp./time | Hybridization temp./time | Sample* | Background** | Signal Intensity DNA | PNA |
|---|---|---|---|---|---|---|
| PC 40% (III)* | - | 50° C/120 min | Mamacarcinoma<br>Tonsils | + 1-1½<br>+0-3 | 2½<br>3 | 2-2½<br>3 |
| PC 40% (III)* | 82° C/5 min | 45° C/60 min | Mamacarcinoma<br>Tonsils | +0-3<br>+0 | ½-1<br>½ | 2<br>1½ |
| PC 15% (IV) | - | 50° C/120 min | Mamacarcinoma<br>Tonsils | +1<br>+0 | 1-2<br>½ | 3<br>3 |
| PC 15% (IV) | 82° C/5 min | 45° C/60 min | Mamacarcinoma<br>Tonsils | +2-3<br>+1-2 | 2-2½<br>2½ | 2-2½<br>2 |
| GBL 40% (V)* | - | 50° C/120 min | Mamacarcinoma<br>Tonsils | +3<br>+2 | ½-1<br>1½-2 | ½<br>½ |
| GBL 40% (V)* | 82° C/5 min | 45° C/60 min | Mamacarcinoma<br>Tonsils | +3<br>+2 | 2-2½<br>2-2½ | ½<br>½ |
| GBL 15% (VI) | - | 50° C/120 min | Mamacarcinoma<br>Tonsils | +2<br>+1 | 1½-2½<br>1-1½ | 1½-2½<br>2 |
| GBL 15% (VI) | 82° C/5 min | 45° C/60 min | Mamacarcinoma<br>Tonsils | +0-1½<br>+0 | 2-2½<br>2½-3 | 2<br>½ |
| EC 15% (VII) | - | 50° C/120 min | Mamacarcinoma<br>Tonsils | +0<br>+0 | 2½-3<br>3 | 2<br>2½ |
| EC 15% (VII) | 82° C/5 min | 45° C/60 min | Mamacarcinoma<br>Tonsils | +2½<br>+1 | 2½<br>3 | 2½<br>2½ |
| FM 15% (VIII) | - | 50° C/120 min | Mamacarcinoma<br>Tonsils | +0<br>+0 | 0-½<br>0-½ | 2<br>2 |
| FM 15% (VIII) | 82° C/5 min | 45° C/60 min | Mamacarcinoma<br>Tonsils | +0<br>+0 | ½<br>½ | 2½-3<br>1½ |
| *The top phase was used. | | | | | | |

These results show that EC, PC, SL and GBL eliminate the need for conventional denaturation of the probe and the sample DNA. In addition, these polar aprotic solvents enable faster hybridizations than the long established gold standard hybridization buffer containing formamide (FISH Probe composition VIII).

## Claims

**1.** A method of hybridizing nucleic acid sequences in situ in a sample on a slide without a denaturation step and on an automated system comprising:

- providing a first nucleic acid sequence,
- providing a second nucleic acid sequence,
- providing a hybridization composition comprising an effective amount of at least one polar aprotic solvent, and
- combining the first and the second nucleic acid sequence and the hybridization composition on the automated system for at least a time period sufficient to hybridize the first and second nucleic acid sequences,

wherein the polar aprotic solvent is not dimethyl sulfoxide (DMSO).

**2.** A method of hybridizing nucleic acid sequences in situ in a sample on a slide without a denaturation step and on an automated system comprising:

- providing a first nucleic acid sequence, and
- applying a hybridization composition comprising a second nucleic acid sequence and an effective amount of at least one polar aprotic solvent for at least a time period sufficient to hybridize the first and second nucleic acid sequences,

wherein the polar aprotic solvent is not dimethyl sulfoxide (DMSO).

**3.** The method according to claims 1 or 2, wherein the first nucleic acid sequence is in a biological sample.

**4.** The method according to any of claims 1-3, wherein the first nucleic acid sequence is a single stranded sequence and the second nucleic acid sequence is a double stranded sequence or wherein the first nucleic acid sequence is a double stranded sequence in a biological sample and the second nucleic acid sequence is a single stranded sequence.

**5.** The method according to any of claims 1-4, wherein a sufficient amount of energy to hybridize the first and second nucleic acids is provided.

**6.** The method according to any one of claims 1-5, wherein the step of hybridization takes less than 8 hours, for example less than 1 hour, or for example less than 30 minutes, or for example less than 15 minutes, or for example less than 5 minutes.

**7.** The method according to any one of claims 1-6, wherein the concentration of polar aprotic solvent is 5% to 10% (v/v) or 10% to 20% (v/v).

**8.** The method according to any one of claims 1-7, wherein the polar aprotic solvent in the hybridization composition has a dispersion solubility parameter between 17.7 to 22.0 $MPa^{1/2}$, a polar solubility parameter between 13 to 23 $Mpa^{1/2}$, and a hydrogen bonding solubility parameter between 3 to 13 $MPa^{1/2}$.

**9.** The method according to any one of claims 1-8, wherein the polar aprotic solvent in the hybridization composition is selected from the group consisting of:

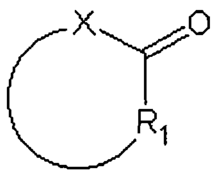 , 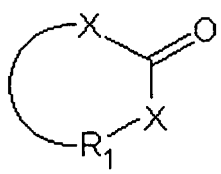 , 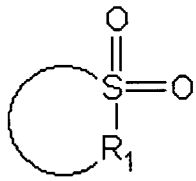 , 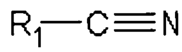

where X is O and R1 is alkyldiyl, and

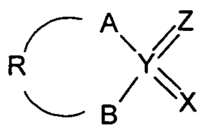

where X is optional and if present, is chosen from O or S,
where Z is optional and if present, is chosen from O or S,
where A and B independently are O or N or S or part of the alkyldiyl or a primary amine,
where R is alkyldiyl, and
where Y is O or S or C.

**10.** The method according to any one of claims 1-9, wherein the polar aprotic solvent in the hybridization composition is selected from the group consisting of:

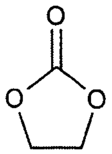 , 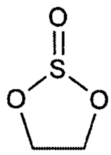 , 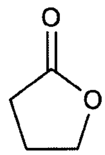 , 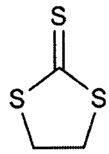 , and 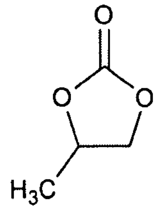

**11.** The method according to any one of claims 1-9, wherein the polar aprotic solvent in the hybridization composition is:

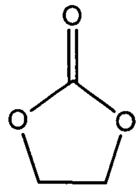 .

**12.** The method according to any one of claims 1-9, wherein the polar aprotic solvent in the hybridization composition is selected from the group consisting of ethylene carbonate, sulfolane, gamma-butyrolactone, propylene carbonate, ethylene trithiocarbonate, glycol sulfite/ethylene sulfite, delta-valerolactam (2-piperidone), and tetrahydrothiophene 1-oxide.

**13.** The method according to any one of claims 1-9, wherein the hybridization composition comprises dextran sulfate and NaCl and/or phosphate buffer.

**14.** The method according to claim 13, wherein the dextran sulfate is present at a concentration of 5% to 40%, the NaCl is present at a concentration of 0 mM to 1200 mM, and/or the phosphate buffer is present at a concentration of 0 mM to 50 mM.

**15.** The method according to any one of claims 1-9, wherein the hybridization composition comprises 15% of at least one polar aprotic solvent, 20% dextran sulfate, 600 mM NaCl, and 10 mM phosphate buffer or the hybridization composition comprises 15% of at least one polar aprotic solvent, 20% dextran sulfate, 600 mM NaCl, and 10 mM citric acid buffer pH 6.2.

## Patentansprüche

**1.** Ein Verfahren zur in situ Hybridisierung von Nukleinsäuresequenzen in einer Probe auf einem Objektträger ohne einen Denaturierungsschritt und auf einem automatisierten System, umfassend:

- Bereitstellung einer ersten Nukleinsäuresequenz,
- Bereitstellung einer zweiten Nukleinsäuresequenz,
- Bereitstellen einer Hybridisierungs-Zusammensetzung, welche eine wirksame Menge von mindestens einem polaren aprotischen Lösungsmittel umfasst, und
- Vereinen der ersten und der zweiten Nukleinsäuresequenz und der Hybridisierungs-Zusammensetzung auf dem automatisierten System für mindestens eine Zeitdauer, die ausreicht um die erste und zweite Nukleinsäuresequenzen zu hybridisieren, wobei das polare aprotische Lösungsmittel nicht Dimethylsulfoxid (DMSO) ist.

**2.** Ein Verfahren zur in situ Hybridisierung von Nukleinsäuresequenzen in einer Probe auf einem Objektträger ohne einen Denaturierungsschritt und auf einem automatisierten System, umfassend:

- Bereitstellung einer ersten Nukleinsäuresequenz,
- Aufbringen einer Hybridisierungs-Zusammensetzung, welche eine zweite Nukleinsäuresequenz und eine wirksame Menge von mindestens einem polaren aprotischen Lösungsmittel umfasst, für mindestens eine Zeitdauer,

die ausreicht um die erste und zweite Nukleinsäuresequenzen zu hybridisieren,

wobei das polare aprotische Lösungsmittel nicht Dimethylsulfoxid (DMSO) ist.

**3.** Das Verfahren nach Anspruch 1 oder 2, wobei die erste Nukleinsäuresequenz in einer biologischen Probe ist.

**4.** Das Verfahren nach einem der Ansprüche 1-3, wobei die erste Nukleinsäuresequenz eine einzelsträngige Sequenz ist und die zweite Nukleinsäuresequenz eine doppelsträngige Sequenz ist, oder wobei die erste Nukleinsäuresequenz eine doppelsträngige Sequenz in einer biologischen Probe ist und die zweite Nukleinsäuresequenz eine einzelsträngige Sequenz ist.

**5.** Das Verfahren nach einem der Ansprüche 1-4, wobei eine ausreichende Menge an Energie zur Verfügung bereitgestellt wird, um die ersten und zweiten Nukleinsäuren zu hybridisieren.

**6.** Das Verfahren nach einem der Ansprüche 1-5, wobei der Schritt der Hybridisierung weniger als 8 Stunden dauert, zum Beispiel weniger als 1 Stunde dauert, oder zum Beispiel weniger als 30 Minuten dauert, oder zum Beispiel weniger als 15 Minuten dauert, oder zum Beispiel weniger als 5 Minuten dauert.

**7.** Das Verfahren nach einem der Ansprüche 1-6, wobei die Konzentration des polaren aprotischen Lösungsmittels bei 5% bis 10% (v / v). oder bei 10% bis 20% (v / v) liegt.

**8.** Das Verfahren nach einem der Ansprüche 1-7, wobei das polare aprotische Lösungsmittel in der Hybridisierungs-Zusammensetzung einen Dispersion Löslichkeitsparameter zwischen 17,7 bis 22,0 $MPa^{1/2}$, einen polaren Löslichkeitsparameter zwischen 13 bis 23 $MPa^{1/2}$, und einen Wasserstoffbindung-Löslichkeitsparameter von 3 bis 13 $MPa^{1/2}$ besitzt.

**9.** Das Verfahren nach einem der Ansprüche 1-8, wobei das polare aprotische Lösungsmittel in der Hybridisierungs-Zusammensetzung ausgewählt wird aus der Gruppe bestehend aus:

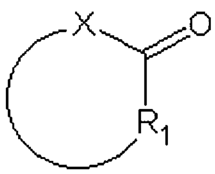 , 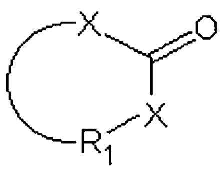 , 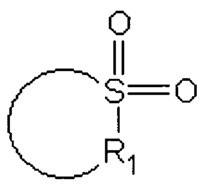 , 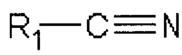

wobei X ist O und R1 ist Alkyldiyl, und

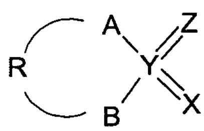

wobei X optional ist und, falls vorhanden, aus O oder S ausgewählt wird,
wobei Z optional ist und, falls vorhanden, aus O oder S gewählt wird,
wobei A und B unabhängig voneinander O oder N oder S oder Teil des Alkyldiyls oder eines primären Amins sind,
wobei R Alkyldiyl ist, und
wobei Y ist O oder S oder C.

**10.** Das Verfahren nach einem der Ansprüche 1-9, wobei das polare aprotische Lösungsmittel in der Hybridisierungs-Zusammensetzung ausgewählt wird aus der Gruppe bestehend aus:

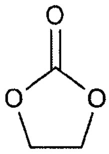 , 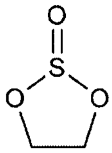 , 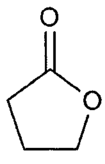 , 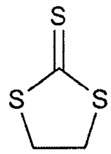 , und 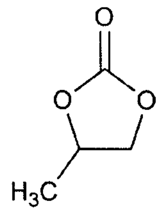

**11.** Das Verfahren nach einem der Ansprüche 1-9, wobei das polare aprotische Lösungsmittel in der Hybridisierungs-Zusammensetzung

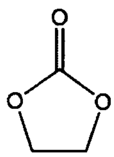

ist.

**12.** Das Verfahren nach einem der Ansprüche 1-9, wobei das polare aprotische Lösungsmittel in der Hybridisierungs-Zusammensetzung ausgewählt wird aus der Gruppe bestehend aus:

Ethylencarbonat, Sulfolan, gamma-Butyrolactone, Propylencarbonat, Ethylentrithiocarbonat, Glycolsulfit/Ethylensulfit, delta-Valerolactam (2-Piperidon), und Tetrahydrothiophen-1-oxid.

**13.** Das Verfahren nach einem der Ansprüche 1-9, wobei die Hybridisierungs-Zusammensetzung Dextransulfat und NaCl und/oder Phosphatpuffer umfasst.

**14.** Das Verfahren nach Anspruch 13, wobei das Dextransulfat in einer Konzentration von 5% bis 40% vorliegt, das NaCl in einer Konzentration von 0 mM bis 1200 mM vorliegt, und/oder der Phosphatpuffer in einer Konzentration von 0 mM bis 50 mM vorliegt.

**15.** Das Verfahren nach einem der Ansprüche 1-9, wobei die Hybridisierungs-Zusammensetzung 15% von mindestens einem polaren aprotischen Lösungsmittel, 20% Dextransulfat, 600 mM NaCl, und 10 mM Phosphatpuffer umfasst, oder die Hybridisierungs-Zusammensetzung umfasst 15% von mindestens einem polaren aprotischen Lösungsmittel, 20% Dextransulfat, 600 mM NaCl, und 10 mM Zitronensäurepuffer pH 6.2.

## Revendications

**1.** Procédé d'hybridation de séquences d'acides nucléiques *in situ* dans un échantillon sur une lame sans étape de dénaturation et sur un système automatisé, comprenant :

- la fourniture d'une première séquence d'acides nucléiques,
- la fourniture d'une deuxième séquence d'acides nucléiques,
- la fourniture d'une composition d'hybridation comprenant une quantité efficace d'au moins un solvant aprotique polaire, et
- la combinaison des première et deuxième séquences d'acides nucléiques et de la composition d'hybridation sur le système automatisé au moins durant une période suffisante pour hybrider les première et deuxième séquences d'acides nucléiques,

dans lequel le solvant aprotique polaire n'est pas le diméthylsulfoxyde (DMSO).

**2.** Procédé d'hybridation de séquences d'acides nucléiques *in situ* dans un échantillon sur une lame sans étape de dénaturation et sur un système automatisé, comprenant :

- la fourniture d'une première séquence d'acides nucléiques, et
- l'application d'une composition d'hybridation comprenant une deuxième séquence d'acides nucléiques et une quantité efficace d'au moins un solvant aprotique polaire au moins durant une période suffisante pour hybrider les première et deuxième séquences d'acides nucléiques,

dans lequel le solvant aprotique polaire n'est pas le diméthylsulfoxyde (DMSO).

**3.** Procédé selon la revendication 1 ou 2, dans lequel la première séquence d'acides nucléiques se trouve dans un échantillon biologique.

**4.** Procédé selon l'une quelconque des revendications 1 à 3, dans lequel la première séquence d'acides nucléiques est une séquence à simple brin et la deuxième séquence d'acides nucléiques est une séquence à double brin, ou dans lequel la première séquence d'acides nucléiques est une séquence à double brin dans un échantillon biologique et la deuxième séquence d'acides nucléiques est une séquence à simple brin.

**5.** Procédé selon l'une quelconque des revendications 1 à 4, dans lequel une quantité d'énergie suffisante est fournie pour hybrider les premier et deuxième acides nucléiques.

**6.** Procédé selon l'une quelconque des revendications 1 à 5, dans lequel l'étape d'hybridation dure moins de 8 heures, par exemple moins d'une heure, ou par exemple moins de 30 minutes, ou par exemple moins de 15 minutes, ou par exemple moins de 5 minutes.

**7.** Procédé selon l'une quelconque des revendications 1 à 6, dans lequel la concentration de solvant aprotique polaire est de 5 % à 10 % (v/v) ou de 10 % à 20 % (v/v).

**8.** Procédé selon l'une quelconque des revendications 1 à 7, dans lequel le solvant aprotique polaire dans la composition d'hybridation présente un paramètre de solubilité en dispersion compris entre 17,7 et 22,0 $MPa^{1/2}$, un paramètre de solubilité polaire compris entre 13 et 23 $MPa^{1/2}$, et un paramètre de solubilité par liaison hydrogène compris entre 3 et 13 $MPa^{1/2}$.

**9.** Procédé selon l'une quelconque des revendications 1 à 8, dans lequel le solvant aprotique polaire dans la composition d'hybridation est sélectionné parmi le groupe constitué par :

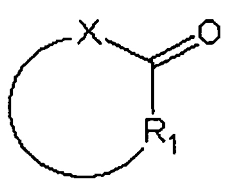 , 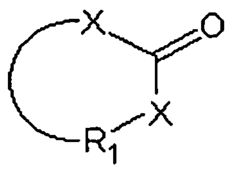 , 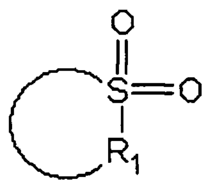 , 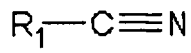

où X est O et R1 est un groupe alkyldiyle, et

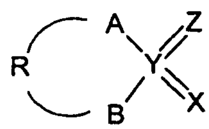

où X est optionnel, et est choisi parmi O et S s'il est présent,
où Z est optionnel, et est choisi parmi O et S s'il est présent,
où A et B sont indépendamment soit O, N, S, une partie de l'alkyldiyle ou une amine primaire,
où R est un alkyldiyle, et
où Y est soit O, S ou C.

**10.** Procédé selon l'une quelconque des revendications 1 à 9, dans lequel le solvant aprotique polaire dans la composition d'hybridation est sélectionné parmi le groupe constitué par :

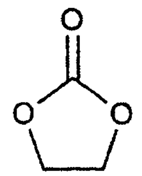 , 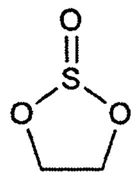 , 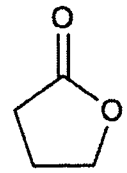 , 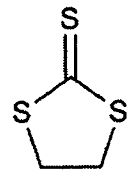 , et 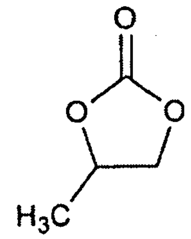

11. Procédé selon l'une quelconque des revendications 1 à 9, dans lequel le solvant aprotique polaire dans la composition d'hybridation est

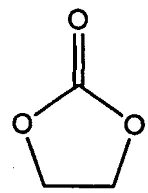.

12. Procédé selon l'une quelconque des revendications 1 à 9, dans lequel le solvant aprotique polaire dans la composition d'hybridation est sélectionné parmi le groupe constitué par le carbonate d'éthylène, le sulfolane, la gamma-butyrolactone, le carbonate de propylène, le trithiocarbonate d'éthylène, le sulfite de glycol / sulfite d'éthylène, le delta-valérolactame (2-pipéridone), et le 1-oxyde de tétrahydrothiophène.

13. Procédé selon l'une quelconque des revendications 1 à 9, dans lequel la composition d'hybridation comprend du sulfate de dextrane et du NaCl et/ou un tampon phosphate.

14. Procédé selon la revendication 13, dans lequel le sulfate de dextrane est présent à une concentration de 5 % à 40 %, le NaCl est présent à une concentration de 0 mM à 1200 mM, et/ou le tampon phosphate est présent à une concentration de 0 mM à 50 mM.

15. Procédé selon l'une quelconque des revendications 1 à 9, dans lequel la composition d'hybridation comprend 15% d'au moins un solvant aprotique polaire, 20 % de sulfate de dextrane, 600 mM de NaCl, et 10 mM de tampon phosphate, ou la composition d'hybridation comprend 15 % d'au moins d'un solvant aprotique polaire, 20 % de sulfate de dextrane, 600 mM de NaCl, et 10 mM de tampon acide citrique à un pH de 6,2.

Figure 1.

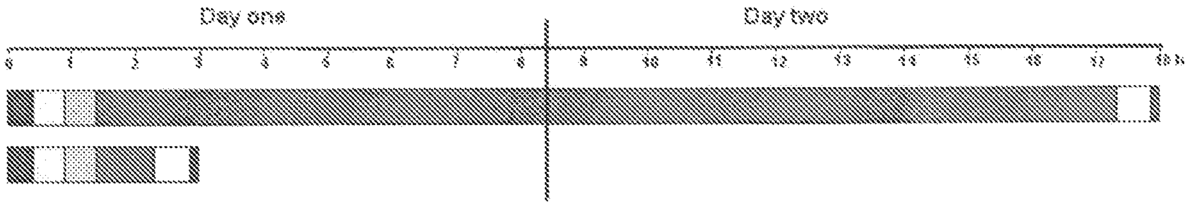

Figure 2.

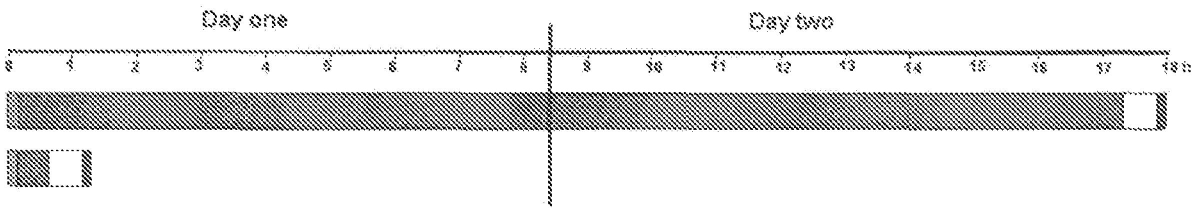

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61265966 A
- US 5539082 A
- US 5527675 A
- US 5623049 A
- US 5714331 A
- US 5718262 A
- US 5736336 A
- US 5773571 A
- US 5766855 A
- US 5786461 A
- US 5837459 A
- US 5891625 A
- US 5972610 A
- US 5986053 A
- US 6107470 A
- US 6201103 B
- US 6228982 B
- US 6357163 B
- WO 9604000 A
- US 0230573 W
- US 20050266459 A
- US 7105294 B
- WO 9914226 A
- US 6730474 B
- DK 2008000430 W
- US 031514 A


### Non-patent literature cited in the description


- **KURRECK.** *Eur. J. Biochem.,* 2003, vol. 270, 1628-44
- **MCCLELLAN A.L.** Tables of Experimental Dipole Moments. W.H. Freeman, 1963
- **SAMBROOK et al.** Molecular Cloning A Laboratory Manual. Cold Spring Harbor Laboratory Press, 1989
- **AUSUBEL et al.** Current Protocols in Molecular Biology. John Wiley & Sons, Inc, 1998
- **POWELL RD et al.** Metallographic in situ hybridization. *Hum. Pathol.,* 2007, vol. 38, 1145-59
- PNA Suppression Method Combined with Fluorescence In Situ Hybridisation (FISH) Technique. **KIRSTEN VANG NIELSEN et al.** PRINS and PNA Technologies in Chromosomal Investigation. Nova Science Publishers, Inc, 2006